(19) Europäisches Patentamt
European Patent Office
Office européen des brevets

(11) Veröffentlichungsnummer: **0 493 670 B1**

(12) **EUROPÄISCHE PATENTSCHRIFT**

(45) Veröffentlichungstag der Patentschrift: **15.02.95**

(51) Int. Cl.$^{6}$: **A01N 43/40**

(21) Anmeldenummer: **91119902.4**

(22) Anmeldetag: **22.11.91**

# (54) Mittel zum Schutz von Pflanzen gegen Befall durch Mikroorganismen.

(30) Priorität: **05.12.90 DE 4038721**

(43) Veröffentlichungstag der Anmeldung:
**08.07.92 Patentblatt 92/28**

(45) Bekanntmachung des Hinweises auf die Patenterteilung:
**15.02.95 Patentblatt 95/07**

(84) Benannte Vertragsstaaten:
**BE CH DE ES FR GB IT LI NL**

(56) Entgegenhaltungen:
**EP-A- 0 268 775**
**WO-A-84/02059**
**US-A- 3 285 926**

**CHEMICAL ABSTRACTS, Band 109, Nr. 19, 7. November 1988, Seite 635,Zusammenfassung Nr. 169434z, Columbus, Ohio, US; E. TYIHAK et al.: "The levelof trigonelline and other quaternary ammonium compounds in tomato leaves in**

**ratio to the changing nitrogen supply",& PLANT SOIL 1988, 109(2), 285-7**

(73) Patentinhaber: **BAYER AG**

**D-51368 Leverkusen (DE)**

(72) Erfinder: **Lunkenheimer, Winfried, Dr.**
**Funckstrasse 49**
**W-5600 Wuppertal (DE)**
Erfinder: **Wollweber, Detlef, Dr.**
**Paul-Ehrlich-Strasse 17**
**W-5600 Wuppertal (DE)**
Erfinder: **Dehne, Heinz-Wilhelm, Dr.**
**Krischer Strasse 81**
**W-4019 Monheim (DE)**
Erfinder: **Dutzmann, Stefan, Dr.**
**Kosenberg 10**
**W-4010 Hilden (DE)**
Erfinder: **Hänssler, Gerd, Dr.**
**Am Arenzberg 58a**
**W-5090 Leverkusen 3 (DE)**
Erfinder: **Mauler-Machnik, Astrid, Dr.**
**Neuenkamper Weg 46a**
**W-5653 Leichlingen (DE)**
Erfinder: **Schulz, Uta, Dr.**
**Metzholz 38**
**W-5653 Leichlingen (DE)**

**EP 0 493 670 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft die Verwendung von teilweise bekannten Pyridinium-Betainen zur Erzeugung von Resistenz in Pflanzen gegen Befall durch unerwünschte Mikroorganismen.

Es ist bereits bekannt, daß sich bestimmte Isonicotincarbonsäure-Derivate zur Erzeugung von Resistenz in Pflanzen gegen Befall durch phytopathogene Mikroorganismen einsetzen lassen (vgl. EP-A 0 268 775). So kann zum Beispiel der 2,6-Dichlor-isonicotinsäure-methylester für den genannten Zweck verwendet werden. Die Wirkung dieses Stoffes ist jedoch nicht immer befriedigend.

Aus der WO-A 84-02059 ist außerdem bekannt, daß sich Trigonellin zur Regulierung des Wachstums und zur Ertragssteigerung bei Leguminosen einsetzen läßt. Eine Verwendung dieses Stoffes zur Induzierung von Resistenz in Pflanzen gegen Krankheitsbefall wird aber nicht erwähnt.

Es wurde nun gefunden, daß die teilweise bekannten Pyridinium-Betaine der Formel

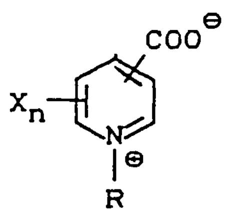

(I)

in welcher

R für Alkyl mit 1 bis 8 Kohlen stoffatomen steht, wobei die Alkylreste einfach bis dreifach, gleichartig oder verschieden substituiert sein können durch Halogen, Hydroxy, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Cyano, Trimethylsilyl, gegebenenfalls durch Halogen und/oder Alkyl mit 1 bis 4 Kohlenstoffatomen substituiertes Cycloalkyl mit 3 bis 6 Kohlenstoffatomen, gegebenenfalls durch Halogen und/oder Alkyl mit 1 bis 4 Kohlenstoffatomen substituiertes Cycloalkenyl mit 5 oder 6 Kohlenstoffatomen, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylthio,
sowie die Reste der Formeln

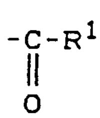 , 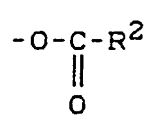, 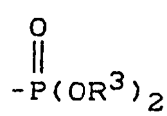 und

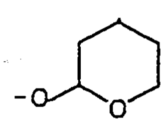 ,

worin

R¹ für Alkyl mit 1 bis 6 Kohlenstoffatomen, gegebenenfalls durch Halogen, Alkyl mit 1 bis 4 Kohlenstoffatomen und/oder Alkoxy mit 1 bis 4 Kohlenstoffatomen substituiertes Phenyl, Hydroxy, Alkoxy mit 1 bis 14 Kohlenstoffatomen, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Benzyloxy, Amino, Alkylamino mit 1 bis 4 Kohlenstoffatomen, Dialkylamino mit 1 bis 4 Kohlenstoffatomen in jeder Alkylgruppe, gegebenenfalls durch Halogen und/oder Alkyl mit 1 bis 4 Kohlenstoffatomen substituiertes Phenyl oder den Rest der Formel

$-NH-CH_2-CO-R^4$ steht, worin

$R^4$ für Hydroxy, Alkoxy mit 1 bis 4 Kohlenstoffatomen, Alkylamino mit 1 bis 4 Kohlenstoffatomen oder für gegebenenfalls durch Halogen und/oder Alkyl mit 1 bis 4 Kohlenstoffatomen substituiertes Phenylamino steht,

$R^2$ für Alkyl mit 1 bis 20 Kohlenstoffatomen, Alkylamino mit 1 bis 4 Kohlenstoffatomen, Dialkylamino mit 1 bis 4 Kohlenstoffatomen in jeder Alkylgruppe oder für gegebenenfalls durch Halogen und/oder Alkyl mit 1 bis 4 Kohlenstoffatomen substituiertes Phenylamino steht und

$R^3$ für Alkyl mit 1 bis 4 Kohlenstoffatomen steht,

oder

R für Alkenyl mit 3 bis 8 Kohlenstoffatomen steht, wobei jeder der Alkenylreste einfach bis dreifach, gleichartig oder verschieden substituiert sein kann durch Halogen, Alkoxy mit 1 bis 4 Kohlenstoffatomen und/oder Alkylthio mit 1 bis 4 Kohlenstoffatomen, oder

R für Alkinyl mit 3 bis 8 Kohlenstoffatomen steht, wobei jeder der Alkinylreste einfach bis dreifach, gleichartig oder verschieden substituiert sein kann durch Halogen, Alkoxy mit 1 bis 4 Kohlenstoffatomen und/oder Alkylthio mit 1 bis 4 Kohlenstoffatomen, oder

R für Aralkyl mit 6 bis 10 Kohlenstoffatomen im Arylteil und 1 bis 4 Kohlenstoffatomen im Alkylteil steht, wobei jeder dieser Reste im Arylteil einfach bis dreifach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R für Aralkenyl mit 6 bis 10 Kohlenstoffatomen im Arylteil und 2 bis 4 Kohlenstoffatomen im Alkenylteil steht, wobei jeder dieser Reste im Arylteil einfach bis dreifach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R für Aralkinyl mit 6 bis 10 Kohlenstoffatomen im Arylteil und 2 bis 4 Kohlenstoffatomen im Alkinylteil steht, wobei jeder dieser Reste im Arylteil einfach bis dreifach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R steht vorzugsweise für gegebenenfalls einfach bis dreifach, gleichartig oder verschieden durch Alkyl mit 1 bis 4 Kohlenstoffatomen substituiertes Cycloalkyl mit 3 bis 8 Kohlenstoffatomen steht, oder

R für gegebenenfalls einfach bis dreifach, gleichartig oder verschieden durch Alkyl mit 1 bis 4 Kohlenstoffatomen substituiertes Cycloalkenyl mit 5 bis 8 Kohlenstoffatomen steht, oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Nitro und/oder Cyano substituiertes Heterocyclylalkyl mit 1 bis 4 Kohlenstoffatomen im Alkylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,

oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder

verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Nitro und/oder Cyano substituiertes Heterocyclylalkenyl mit 3 bis 6 Kohlenstoffatomen im Alkenylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,
oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen, Nitro und/oder Cyano substituiertes Heterocyclylalkinyl mit 3 bis 6 Kohlenstoffatomen im Alkinylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,

X für Fluor, Chlor, Brom, Jod, Alkyl mit 1 bis 8 Kohlenstoffatomen, Halogenalkyl mit 1 bis 4 Kohlenstoffatomen und 1 bis 5 gleichen oder verschiedenen Halogenatomen, Alkoxy mit 1 bis 8 Kohlenstoffatomen, Halogenalkoxy mit 1 bis 4 Kohlenstoffatomen und 1 bis 5 gleichen oder verschiedenen Halogenatomen, Hydroxy, Mercapto, Alkylthio mit 1 bis 8 Kohlenstoffatomen, Halogenalkylthio mit 1 bis 4 Kohlenstoffatomen und 1 bis 5 gleichen oder verschiedenen Halogenatomen, Amino, Alkylamino mit 1 bis 6 Kohlenstoffatomen, Dialkylamino mit 1 bis 6 Kohlenstoffatomen in jeder Alkylgruppe, Phenylamino, Benzylamino oder gegebenenfalls durch Halogen und/oder Alkyl mit 1 bis 4 Kohlenstoffatomen substituiertes Phenyl steht,
und

n für 0, 1, 2, 3 oder 4 steht,
wobei die durch X bezeichneten Substituenten gleichartig oder verschieden sein können, wenn n für 2, 3 oder 4 steht,

sehr gut zur Erzeugung von Resistenz in Pflanzen gegen Befall durch unerwünschte Mikroorganismen verwendbar sind.

Überraschenderweise eignen sich die erfindungsgemäßen Pyridinium-Betaine der Formel (I) besser zur Erzeugung von Resistenz in Pflanzen gegen Befall durch phytopathogene Mikroorganismen als die kontitutionell ähnlichsten Verbindungen, die aus dem Stand der Technik bekannt und für den gleichen Zweck einsetzbar sind. So übertreffen die erfindungsgemäßen Stoffe zum Beispiel den 2,6-Dichlor-isonicotinsäure-methylester bezüglich der resistenzerzeugenden Wirkung in Pflanzen gegen Befall durch unerwünschte Mikroorganismen.

Die erfindungsgemäßen verwendbaren Pyridinium-Betaine sind durch die Formel (I) allgemein definiert.

R steht bevorzugt für Alkyl mit 1 bis 6 Kohlenstoffatomen, wobei die Alkylreste einfach bis dreifach, gleichartig oder verschieden substituiert sein können durch Fluor, Chlor, Brom, Hydroxy, Methoxy, Ethoxy, Methylthio, Ethylthio, Methylsulfinyl, Methylsulfonyl, Cyano, Trimethylsilyl, gegebenenfalls einfach bis dreifach durch Fluor, Chlor und/oder Methyl substituiertes Cycloalkyl mit 3 bis 6 Kohlenstoffatomen, gegebenenfalls einfach bis dreifach durch Fluor, Chlor und/oder Methyl substituiertes Cycloalkenyl mit 5 oder 6 Kohlenstoffatomen, gegebenenfalls durch Fluor, Chlor und/oder Methyl substituiertes Phenoxy, gegebenenfalls durch Fluor, Chlor und/oder Methyl substituiertes Phenylthio, sowie für die Reste der Formeln

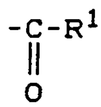, 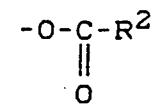, 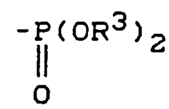 und

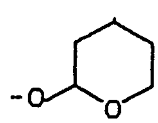,

worin

R¹ für Alkyl mit 1 bis 4 Kohlenstoffatomen, gegebenenfalls durch Fluor, Chlor, Methyl und/oder

Methoxy substituiertes Phenyl, Hydroxy, Alkoxy mit 1 bis 12 Kohlenstoffatomen, gegebenenfalls durch Fluor, Chlor und/oder Methyl substituiertes Phenoxy, gegebenenfalls durch Fluor, Chlor und/oder Methyl substituiertes Benzyloxy, Amino, Methylamino, Ethylamino, Dimethylamino, Diethylamino, gegebenenfalls durch Fluor, Chlor und/oder Methyl substituiertes Phenylamino oder den Rest der Formel

$-NH-CH_2-CO-R^4$ steht, worin

$R^4$ für Hydroxy, Methoxy, Ethoxy, Methylamino, Ethylamino oder für gegebenenfalls durch Fluor, Chlor und/oder Methyl substituiertes Phenylamino steht,

$R^2$ für Alkyl mit 1 bis 20 Kohlenstoffatomen, Methylamino, Ethylamino, Dimethylamino, Diethylamino oder für gegebenenfalls durch Fluor, Chlor und/oder Methyl substituiertes Phenylamino steht und

$R^3$ für Methyl oder Ethyl steht, oder

R steht bevorzugt für Alkenyl mit 3 bis 6 Kohlenstoffatomen, wobei jeder der Alkenylreste einfach bis dreifach, gleichartig oder verschieden substituiert sein kann durch Fluor, Chlor, Methoxy, Ethoxy, Methylthio und/oder Ethylthio,
oder

R steht bevorzugt für Alkinyl mit 3 bis 6 Kohlenstoffatomen, wobei jeder der Alkinylreste einfach bis dreifach, gleichartig oder verschieden substituiert sein kann durch Fluor, Chlor, Methoxy, Ethoxy, Methylthio und/oder Ethylthio, oder

R steht bevorzugt für Phenylalkyl oder Naphthylalkyl mit jeweils 1 oder 2 Kohlenstoffatomen im Alkylteil, wobei jeder dieser Reste im Arylteil einfach bis dreifach, gleichartig oder verschieden substituiert sein kann durch Fluor, Chlor, Brom, Methyl, Ethyl, Halogenalkyl mit 1 oder 2 Kohlenstoffatomen und 1 bis 3 Fluor-, Chlor- und/oder Bromatomen, Methoxy, Ethoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Nitro, Cyano, Phenyl, Phenoxy, Phenylthio, Methoxycarbonyl, Ethoxycarbonyl, Methylcarbonyl und/oder Ethylcarbonyl, oder

R steht bevorzugt für Phenylalkenyl mit 3 oder 4 Kohlenstoffatomen im Alkenylteil, wobei jeder dieser Reste im Phenylteil einfach bis dreifach, gleichartig oder verschieden substituiert sein kann durch Fluor, Chlor, Brom, Methyl, Ethyl, Halogenalkyl mit 1 oder 2 Kohlenstoffatomen und 1 bis 3 Fluor-, Chlor- und/oder Bromatomen, Methoxy, Ethoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Nitro, Cyano, Phenyl, Phenoxy, Phenylthio, Methoxycarbonyl, Ethoxycarbonyl, Methylcarbonyl und/oder Ethylcarbonyl, oder

R steht bevorzugt für Phenylalkinyl mit 3 oder 4 Kohlenstoffatomen im Alkinylteil, wobei jeder dieser Reste im Phenylteil einfach bis dreifach, gleichartig oder verschieden substituiert sein kann durch Fluor, Chlor, Brom, Methyl, Ethyl, Halogenalkyl mit 1 oder 2 Kohlenstoffatomen und 1 bis 3 Fluor-, Chlor- und/oder Bromatomen, Methoxy, Ethoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Nitro, Cyano, Phenyl, Phenoxy, Phenylthio, Methoxycarbonyl, Ethoxycarbonyl, Methylcarbonyl und/oder Ethylcarbonyl, oder

R steht bevorzugt für gegebenenfalls einfach bis dreifach gleichartig oder verschieden durch Methyl, Ethyl, n-Propyl und/oder Isopropyl substituiertes Cycloalkyl mit 3 bis 7 Kohlenstoffatomen, oder

R steht bevorzugt für gegebenenfalls einfach bis dreifach, gleichartig oder verschieden durch Methyl, Ethyl, n-Propyl und/oder Isopropyl substituiertes Cycloalkenyl mit 5 bis 7 Kohlenstoffatomen, oder

R steht bevorzugt für den Rest der Formel

$-(CH_2)_m-Het$, worin

m für 1, 2 oder 3 steht und

Het für einen Rest der Formeln

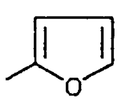 , 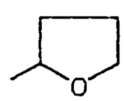 , 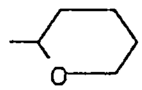 , 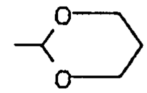 , 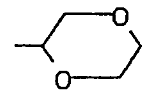 ,

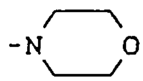 , 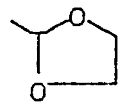 , 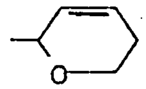 , 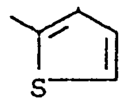 , 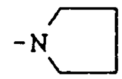 ,

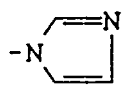 , 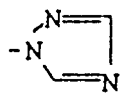 , 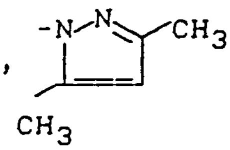 , 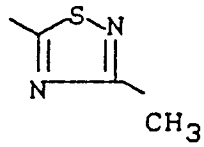 ,

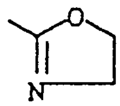 , 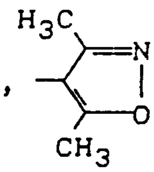 , 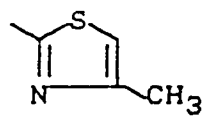 , 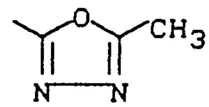 ,

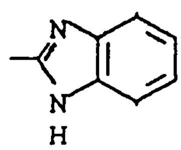 , 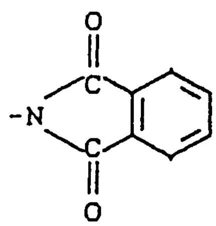 , 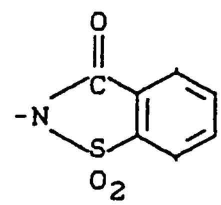 ,

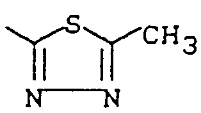 , 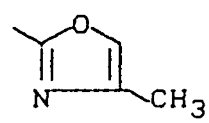 , 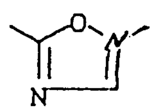 , 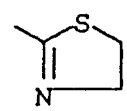 ,

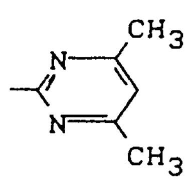 , 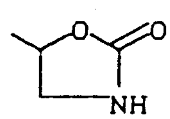 , 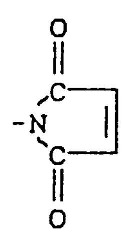 oder

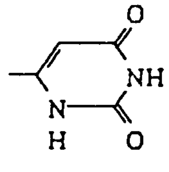

steht,
oder

R steht bevorzugt für einen Rest der Formel

-Z-Het, worin

Z für Alkenylyl mit 3 oder 4 Kohlenstoffatomen oder Alkinylyl mit 3 oder 4 Kohlenstoffatomen steht und

Het für einen Rest der Formeln

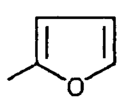 , 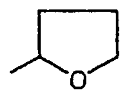 , 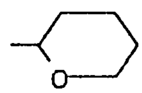 , 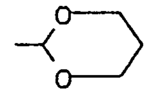 , 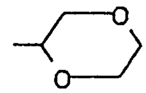 ,

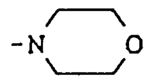 , 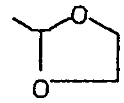 , 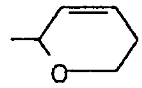 , 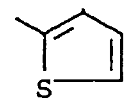 , 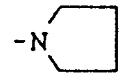 ,

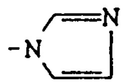 , 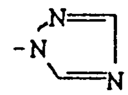 , 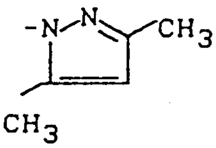 , 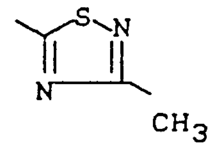 ,

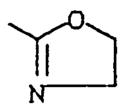 , 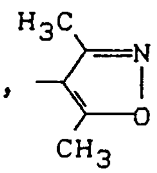 , 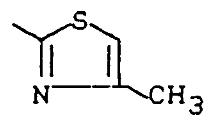 , 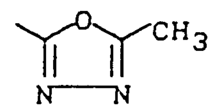 ,

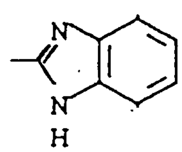 , 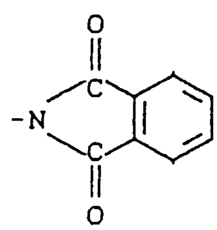 , 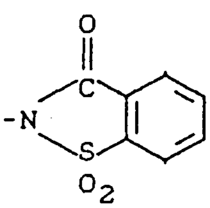 ,

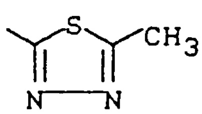 , 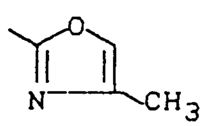 , 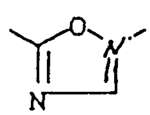 , 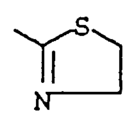 ,

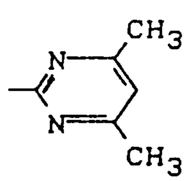 , 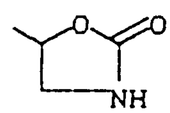 , 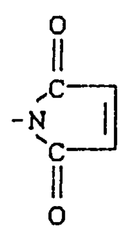 oder

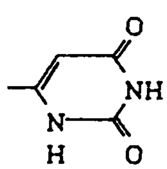

steht.

X steht bevorzugt für Fluor, Chlor, Brom, Iod, Alkyl mit 1 bis 6 Kohlenstoffatomen, Halogenalkyl mit 1 oder 2 Kohlenstoffatomen und 1 bis 3 gleichen oder verschiedenen Fluor-, Chlor- und/oder Bromatomen, Alkoxy mit 1 bis 6 Kohlenstoffatomen, Halogenalkoxy mit 1 oder 2 Kohlenstoffatomen und 1 bis 3 gleichen oder verschiedenen Fluor-, Chlor- und/oder Bromatomen, Hydroxy, Mercapto, Alkylthio mit 1 bis 6 Kohlenstoffatomen, Halogenalkylthio mit 1 oder 2 Kohlenstoffatomen und 1 bis 3 gleichen oder verschiedenen Fluor-, Chlor- und/oder Bromatomen, Amino, Alkylamino mit 1 bis 4 Kohlenstoffatomen, Dialkylamino mit 1 bis 4 Kohlenstoffatomen in jeder Alkylgruppe, Phenylamino, Benzylamino oder für gegebenenfalls einfach oder zweifach, gleichartig oder verschieden durch Fluor, Chlor, Methyl und/oder Ethyl substituiertes Phenyl.

Als Beispiele für erfindungsgemäß verwendbare Pyridinium-Betaine seien die in den folgenden Tabellen aufgeführten Stoffe genannt.

Tabelle 1

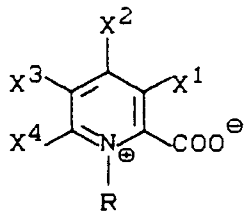

(Ia)

| R | $X^1$ | $X^2$ | $X^3$ | $X^4$ |
|---|---|---|---|---|
| $-C_3H_7$-n | H | H | H | H |
| $-C_4H_9$-n | " | " | " | " |
| $-C_5H_{11}$-n | " | " | " | " |
| $-C_6H_{13}$-n | " | " | " | " |
| $-C_3H_7$-iso | " | " | " | " |
| $-CH_2CF_3$ | " | " | " | " |
| $-CH_2-CH_2-Br$ | " | " | " | " |
| $-CH_2-CN$ | " | " | " | " |
| $-CH_2-CH=CH_2$ | | | | |
| $-CH_2$—C≡C— 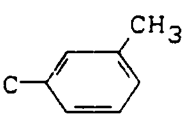 | " | " | " | " |
| $-CH_2-CO$— 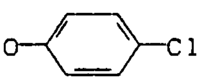 | H | H | H | H |
| $-CH_2-CH_2-SO_2-CH_3$ | " | " | " | " |
| $-CH_2-CH_2-P(O)(OC_2H_5)_2$ | " | " | " | " |
| $-CH_2-COO-C_4H_9$-n | " | " | " | " |
| $-CH_2-COO-C_{10}-H_{21}$ | " | " | " | " |
| $-CH_2-CO-NH-CH_2-CO(NH-CH_3)$ | " | " | " | " |
| $-CH_2-CH_2-O-CO-C_2H_5$ | " | " | " | " |
| $-CH_2-CH_2-O-CO-NH(CH_3)$ | " | " | " | " |
| $-CH_2-CH(OC_2H_5)_2$ | " | " | " | " |

Tabelle 1 (Fortsetzung)

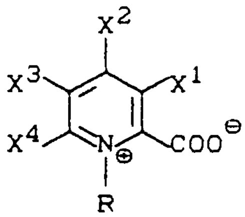

(Ia)

| R | $X^1$ | $X^2$ | $X^3$ | $X^4$ |
|---|---|---|---|---|
| 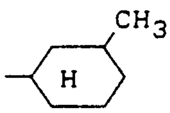 | H | H | H | H |
| 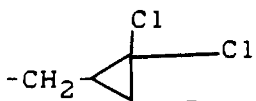 | " | " | " | " |
| 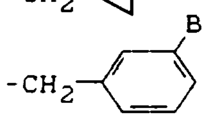 | " | " | " | " |
| 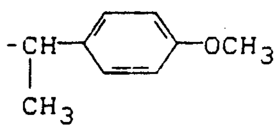 | " | " | " | " |
| 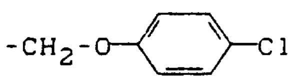 | " | " | " | " |
| 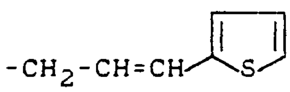 | H | H | H | H |
| 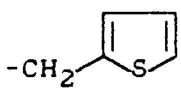 | " | " | " | " |
| 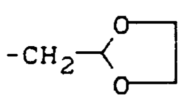 | " | " | " | " |
| 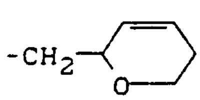 | " | " | " | " |
| 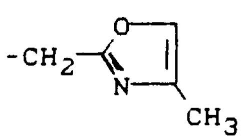 | " | " | " | " |

Tabelle 1 (Fortsetzung)

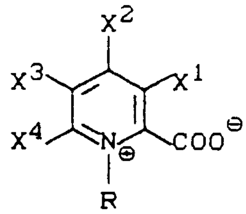

(Ia)

| R | $X^1$ | $X^2$ | $X^3$ | $X^4$ |
|---|---|---|---|---|
| 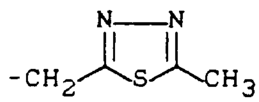 | H | H | H | H |
| 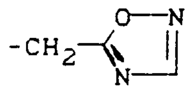 | " | " | " | " |
| 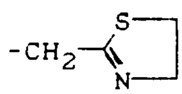 | " | " | " | " |
| 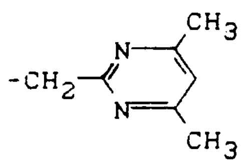 | " | " | " | " |
| 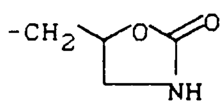 | " | " | " | " |
| 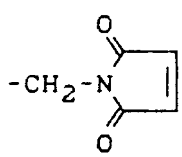 | H | H | H | H |
| 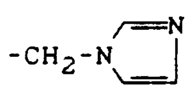 | " | " | " | " |
| 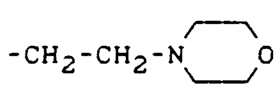 | " | " | " | " |
| 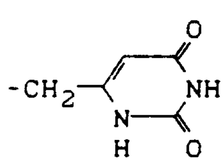 | " | " | " | " |

Tabelle 1 (Fortsetzung)

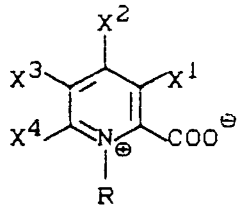

(Ia)

| R | $X^1$ | $X^2$ | $X^3$ | $X^4$ |
|---|---|---|---|---|
| $-CH_2-$ 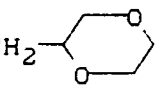 | H | H | H | H |
| $-CH_2-$ 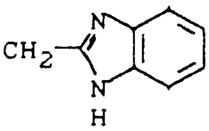 | " | " | " | " |
| $-CH_3$ | $CH_3$ | H | H | H |
| $-CH_3$ | H | $CH_3$ | H | H |
| $-CH_3$ | H | H | $CH_3$ | H |
| $-CH_3$ | H | H | H | $CH_3$ |
| $-CH_3$ | $CH_3$ | $CH_3$ | H | H |
| $-CH_3$ | $CH_3$ | H | $CH_3$ | H |
| $-CH_3$ | $CH_3$ | H | H | $CH_3$ |
| $-CH_3$ | H | $CH_3$ | $CH_3$ | H |
| $-CH_3$ | H | $CH_3$ | H | $CH_3$ |
| $-CH_3$ | H | H | $CH_3$ | $CH_3$ |
| $-CH_3$ | $CH_3$ | $CH_3$ | H | $CH_3$ |
| $-CH_3$ | $CH_3$ | $CH_3$ | $CH_3$ | $CH_3$ |
| $-CH_3$ | $C_2H_5$ | H | H | H |

Tabelle 1 (Fortsetzung)

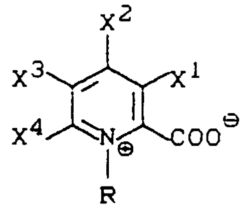

(Ia)

| R | $X^1$ | $X^2$ | $X^3$ | $X^4$ |
|---|---|---|---|---|
| $-CH_3$ | Cl | H | H | H |
| $-CH_3$ | H | Cl | H | H |
| $-CH_3$ | H | H | Cl | H |
| $-CH_3$ | H | H | H | Cl |
| $-CH_3$ | Cl | Cl | H | H |
| $-CH_3$ | Cl | H | Cl | H |
| $-CH_3$ | H | Cl | H | Cl |
| $-CH_3$ | H | H | Cl | Cl |
| $-CH_3$ | Br | H | H | H |
| $-CH_3$ | H | H | Br | H |
| $-CH_3$ | F | H | H | H |
| $-CH_3$ | H | H | H | F |
| $-CH_3$ | H | H | F | H |
| $-CH_3$ | $CH_3$ | $CH_3$ | Cl | $CH_3$ |
| $-CH_3$ | $CH_3$ | $CF_3$ | H | $CF_3$ |
| $-CH_3$ | H | H | H | $CF_3$ |

Tabelle 1 (Fortsetzung)

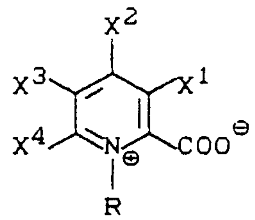

(Ia)

| R | $X^1$ | $X^2$ | $X^3$ | $X^4$ |
|---|---|---|---|---|
| $-CH_3$ | $CF_3$ | $CH_3$ | $CH_3$ | $CH_3$ |
| $-CH_3$ | $CH_3$ | $C_2F_5$ | H | $C_2F_5$ |
| $-CH_3$ | $OCF_3$ | $CH_3$ | H | $CH_3$ |
| $-CH_3$ | $CH_3$ | $OCF_3$ | H | $OCF_3$ |
| $-CH_3$ | $SCF_3$ | H | H | H |
| $-CH_3$ | H | H | OH | H |
| $-CH_3$ | H | OH | H | H |
| $-CH_3$ | OH | H | H | H |
| $-CH_3$ | H | SH | H | H |
| $-CH_3$ | H | H | SH | H |
| $-CH_3$ | SH | H | H | H |
| $-CH_3$ | $NH_2$ | H | H | H |
| $-CH_3$ | H | H | $NH_2$ | H |
| $-CH_3$ | H | H | $OCH_3$ | H |
| $-CH_3$ | H | $OCH_3$ | H | H |
| $-CH_3$ | $OCH_3$ | H | H | H |
| $-CH_3$ | $OC_2H_5$ | H | H | H |
| $-CH_3$ | H | $SCH_3$ | H | H |
| $-CH_3$ | H | H | $SCH_3$ | H |

Tabelle 1 (Fortsetzung)

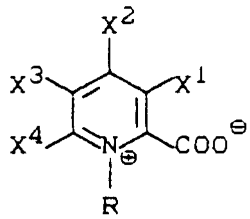

(Ia)

| R | $X^1$ | $X^2$ | $X^3$ | $X^4$ |
|---|---|---|---|---|
| $-CH_3$ | $SC_2H_5$ | H | H | H |
| $-CH_3$ | $NH-CH_3$ | H | H | H |
| $-CH_3$ | $N(CH_3)_2$ | H | H | H |
| $-CH_3$ | H | H | $N(C_2H_5)_2$ | H |
| $-CH_3$ | H | H | H | NH 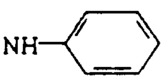 |
| $-CH_3$ | $NH-CH_2$ 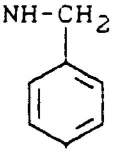 | H | H | H |
| $-CH_3$ | H | 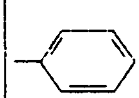 | H | 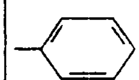 |
| $-CH_3$ | 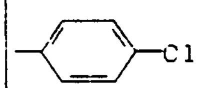 | $CH_3$ | H | $CH_3$ |
| $-CH_3$ | 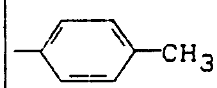 | H | H | 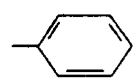 |
| $-CH_3$ | 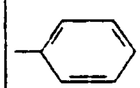 | $CH_3$ | H | 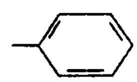 |
| $-C_2H_5$ | $CH_3$ | H | H | H |
| $-C_2H_5$ | H | $CH_3$ | H | H |
| $-C_2H_5$ | H | H | $CH_3$ | H |

Tabelle 1 (Fortsetzung)

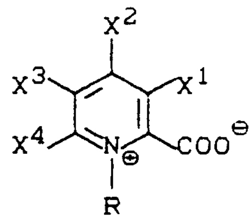

(Ia)

| R | $X^1$ | $X^2$ | $X^3$ | $X^4$ |
|---|---|---|---|---|
| $-C_2H_5$ | H | H | H | $CH_3$ |
| $-C_2H_5$ | $CH_3$ | $CH_3$ | H | H |
| $-C_2H_5$ | $CH_3$ | H | $CH_3$ | H |
| $-C_2H_5$ | $CH_3$ | H | H | $CH_3$ |
| $-C_2H_5$ | H | $CH_3$ | $CH_3$ | H |
| $-C_2H_5$ | H | $CH_3$ | H | $CH_3$ |
| $-C_2H_5$ | H | H | $CH_3$ | $CH_3$ |
| $-C_2H_5$ | $CH_3$ | $CH_3$ | H | $CH_3$ |
| $-C_2H_5$ | $CH_3$ | $CH_3$ | $CH_3$ | $CH_3$ |
| $-C_2H_5$ | $C_2H_5$ | H | H | H |
| $-C_2H_5$ | H | H | H | $C_2H_5$ |
| $-C_2H_5$ | H | H | H | $C_3H_7$-n |
| $-C_2H_5$ | Cl | H | H | H |

Tabelle 1 (Fortsetzung)

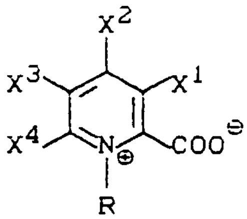

(Ia)

| R | $X^1$ | $X^2$ | $X^3$ | $X^4$ |
|---|---|---|---|---|
| $-C_2H_5$ | H | Cl | H | H |
| $-C_2H_5$ | H | H | Cl | H |
| $-C_2H_5$ | H | H | H | Cl |
| $-C_2H_5$ | Cl | Cl | H | H |
| $-C_2H_5$ | Cl | H | Cl | H |
| $-C_2H_5$ | H | Cl | H | Cl |
| $-C_2H_5$ | H | H | Cl | Cl |
| $-C_2H_5$ | Br | H | H | H |
| $-C_2H_5$ | H | H | Br | H |
| $-C_2H_5$ | F | H | H | H |
| $-C_2H_5$ | H | H | H | F |
| $-C_2H_5$ | H | H | F | H |
| $-C_2H_5$ | $CH_3$ | $CH_3$ | Cl | $CH_3$ |
| $-C_2H_5$ | $CH_3$ | $CF_3$ | H | $CF_3$ |
| $-C_2H_5$ | H | H | H | $CF_3$ |
| $-C_2H_5$ | $CF_3$ | $CH_3$ | $CH_3$ | $CH_3$ |
| $-C_2H_5$ | $CH_3$ | $C_2F_5$ | H | $C_2F_5$ |
| $-C_2H_5$ | $OCF_3$ | $CH_3$ | H | $CH_3$ |
| $-C_2H_5$ | $CH_3$ | $OCF_3$ | H | $OCF_3$ |
| $-C_2H_5$ | $SCF_3$ | H | H | H |

Tabelle 1 (Fortsetzung)

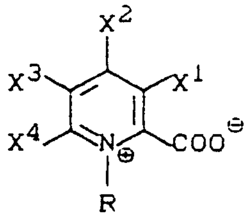

(Ia)

| R | $X^1$ | $X^2$ | $X^3$ | $X^4$ |
|---|---|---|---|---|
| $-C_2H_5$ | H | H | OH | H |
| $-C_2H_5$ | H | OH | H | H |
| $-C_2H_5$ | OH | H | H | H |
| $-C_2H_5$ | H | SH | H | H |
| $-C_2H_5$ | H | H | SH | H |
| $-C_2H_5$ | SH | H | H | H |
| $-C_2H_5$ | $NH_2$ | H | H | H |
| $-C_2H_5$ | H | H | $NH_2$ | H |
| $-C_2H_5$ | H | H | $OCH_3$ | H |
| $-C_2H_5$ | H | $OCH_3$ | H | H |

Tabelle 1 (Fortsetzung)

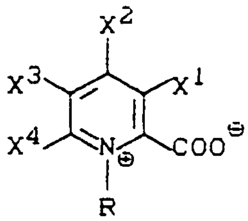

(Ia)

| R | $X^1$ | $X^2$ | $X^3$ | $X^4$ |
|---|---|---|---|---|
| $-C_2H_5$ | $OCH_3$ | H | H | H |
| $-C_2H_5$ | $OC_2H_5$ | H | H | H |
| $-C_2H_5$ | H | $SCH_3$ | H | H |
| $-C_2H_5$ | H | H | $SCH_3$ | H |
| $-C_2H_5$ | $SC_2H_5$ | H | H | H |
| $-C_2H_5$ | $NH-CH_3$ | H | H | H |
| $-C_2H_5$ | $N(CH_3)_2$ | H | H | H |
| $-C_2H_5$ | H | H | $N(C_2H_5)_2$ | H |
| $-C_2H_5$ | H | H | H | NH– 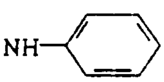 |

Tabelle 1 (Fortsetzung)

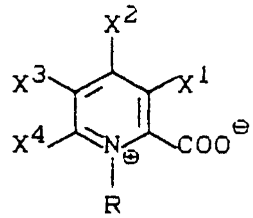

(Ia)

| R | $X^1$ | $X^2$ | $X^3$ | $X^4$ |
|---|---|---|---|---|
| $-C_2H_5$ | 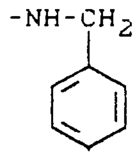 | H | H | H |
| $-C_2H_5$ | H | 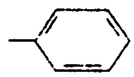 | H | 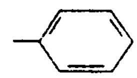 |
| $-C_2H_5$ | 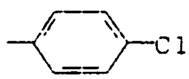 | $CH_3$ | H | $CH_3$ |
| $-C_2H_5$ | 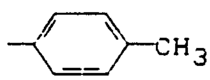 | H | H | 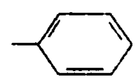 |
| $-C_2H_5$ | 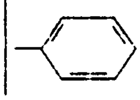 | $CH_3$ | H | 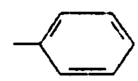 |

Tabelle 1 (Fortsetzung)

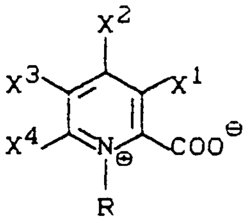

(Ia)

| R | $X^1$ | $X^2$ | $X^3$ | $X^4$ |
|---|---|---|---|---|
| $-C_3H_7-n$ | $CH_3$ | H | H | H |
| $-C_4H_9-n$ | H | $CH_3$ | H | H |
| $-CH_2-CH=CH_2$ | H | H | $CH_3$ | H |
| $-CH_2-COO-C_2H_5$ | H | H | H | $CH_3$ |
| $-CH_2-CO-$ 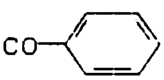 | $CH_3$ | $CH_3$ | H | H |
| $-CH_2-CH_2-OH$ | $CH_3$ | H | $CH_3$ | H |
| $-CH_2-CH_2-O-\underset{\substack{\\| \\ O}}{C}-CH_3$ | $CH_3$ | H | H | $CH_3$ |

Tabelle 1 (Fortsetzung)

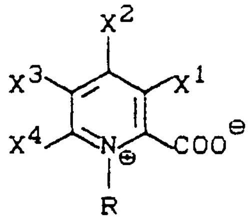

(Ia)

| R | $X^1$ | $X^2$ | $X^3$ | $X^4$ |
|---|---|---|---|---|
| $-CH_2-$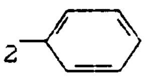 | H | $CH_3$ | $CH_3$ | H |
| $-CH_2-$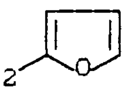 | H | $CH_3$ | H | $CH_3$ |
| $-CH_2-O-C_2H_5$ | H | H | $CH_3$ | $CH_3$ |
| $-CH_2-$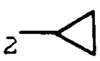 | $CH_3$ | $CH_3$ | H | $CH_3$ |
| $-CH_2-S-$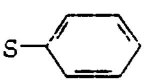 | $CH_3$ | $CH_3$ | $CH_3$ | $CH_3$ |

Tabelle 1 (Fortsetzung)

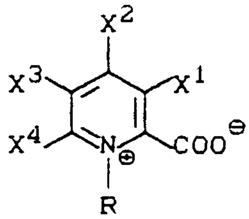

(Ia)

| R | $X^1$ | $X^2$ | $X^3$ | $X^4$ |
|---|---|---|---|---|
| $-CH_2-CH_2-\underset{\underset{O}{\|}}{C}-NH-CH_3$ | $C_2H_5$ | H | H | H |
| $-CH_2-C{\equiv}CH$ | H | H | H | $C_2H_5$ |
| $-CH_2-$ 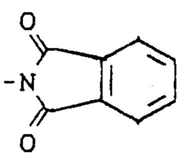 | H | H | H | $C_3H_7$-n |
| $-CH_2-$ 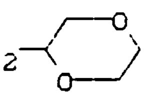 | Cl | H | H | H |
| $-CH_2-CH_2-$ 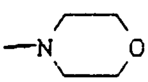 | H | Cl | H | H |
| $-CH_2-CH_2-CH(OC_2H_5)_2$ | H | H | Cl | H |
| $CF_3$ | H | H | H | Cl |

Tabelle 1 (Fortsetzung)

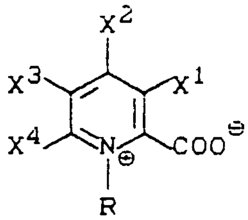

(Ia)

| R | $X^1$ | $X^2$ | $X^3$ | $X^4$ |
|---|---|---|---|---|
| 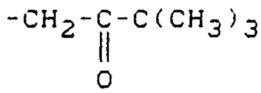 | Cl | Cl | H | H |
| $-CH_2-CH_2-S-CH_3$ | Cl | H | Cl | H |
| $-C_3H_7$-n | H | Cl | H | Cl |
| $-C_6H_{13}$-n | H | H | Cl | Cl |
| $-CH_2-CH_2-O-CH_3$ | Br | H | H | H |
| 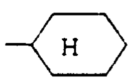 | H | H | Br | H |
| $-CH_2$ 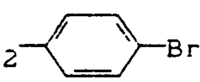 Br | F | H | H | H |
| $-C_3H_7$-n | H | H | F | H |

Tabelle 1 (Fortsetzung)

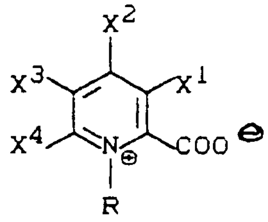

(Ia)

| R | $X^1$ | $X^2$ | $X^3$ | $X^4$ |
|---|---|---|---|---|
| -$CH_2$-$CH_2$-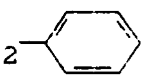 | H | H | H | F |
| -$C_4H_9$-iso | $CH_3$ | $CH_3$ | Cl | $CH_3$ |
| -$CH_2$-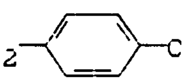-Cl | $CH_3$ | $CF_3$ | H | $CF_3$ |
| -$CH_2$-CO-$CH_3$ | H | H | H | $CF_3$ |
| -$CH_2$ 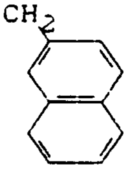 | $CF_3$ | $CH_3$ | $CH_3$ | $CH_3$ |
| -$CH_2$-O-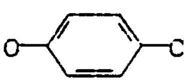-Cl | $CH_3$ | $C_2F_5$ | H | $C_2F_5$ |

Tabelle 1 (Fortsetzung)

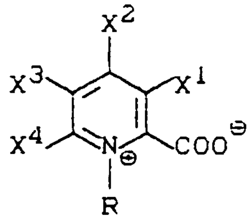

(Ia)

| R | $X^1$ | $X^2$ | $X^3$ | $X^4$ |
|---|---|---|---|---|
| $-C_5H_{11}-n$ | $OCF_3$ | $CH_3$ | H | $CH_3$ |
| $-C_3H_7-n$ | $CH_3$ | $OCF_3$ | H | $OCF_3$ |
| $-CH_2-$ 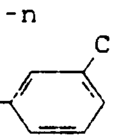 | $SCF_3$ | H | H | H |
| $-CH_2-$ 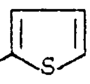 | H | H | OH | H |
| $-CH_2-$ 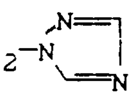 | H | OH | H | H |
| $-C_3H_7-n$ | OH | H | H | H |
| $-C(CH_3)_3$ | H | SH | H | H |

Tabelle 1 (Fortsetzung)

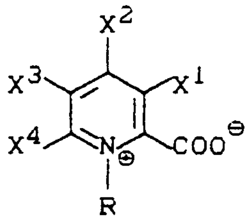

(Ia)

| R | $X^1$ | $X^2$ | $X^3$ | $X^4$ |
|---|---|---|---|---|
| 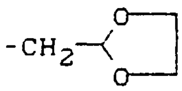 | H | H | SH | H |
| $-CH_2-CH_2-OH$ | SH | H | H | H |
| $-C_3H_7-n$ | $NH_2$ | H | H | H |
| $-C_4H_9-n$ | H | H | $NH_2$ | H |
| 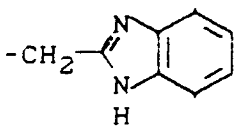 | H | H | $OCH_3$ | H |
| 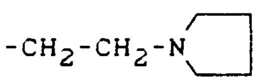 | H | $OCH_3$ | H | H |

Tabelle 1 (Fortsetzung)

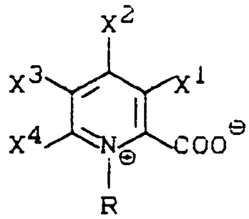

(Ia)

| R | $X^1$ | $X^2$ | $X^3$ | $X^4$ |
|---|---|---|---|---|
| $-CH_2-CN$ | $OCH_3$ | H | H | H |
| $-CH_2-CH_2-S-CH_3$ | $OC_2H_5$ | H | H | H |
| $-CH_2-CH=CH_2$ | H | $SCH_3$ | H | H |
| $-CH_2-CF_3$ | H | H | $SCH_3$ | H |
| $-CH_2-CH_2-\underset{\underset{O}{\Vert}}{C}-NH-CH_3$ | $SC_2H_5$ | H | H | H |
| $-C_5H_{11}-n$ | $NH-CH_3$ | H | H | H |
| $-C_3H_7-n$ | $N(CH_3)_2$ | H | H | H |

Tabelle 1 (Fortsetzung)

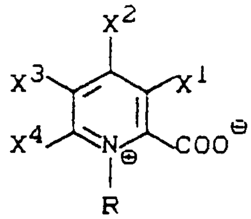

(Ia)

| R | $X^1$ | $X^2$ | $X^3$ | $X^4$ |
|---|---|---|---|---|
| $-CH_2-CH_2-CO-CH_3$ | H | H | $N(C_2H_5)_2$ | H |
| $-CH_2-CH_2-CO-NH_2$ | H | H | H | 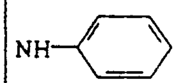 |
| $-CH_2$ 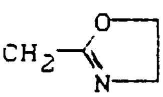 | NH-$CH_2$ 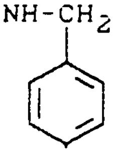 | H | H | H |
| $-C_3H_7$-n | H | 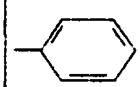 | H | 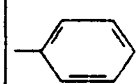 |
| $-C_4H_9$-n | 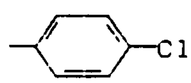 | $CH_3$ | H | $CH_3$ |
| $-CH_2-CH_2-O-CH_3$ | 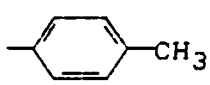 | H | H | 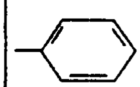 |
| $-C_6H_{13}$-n | 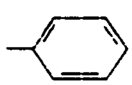 | $CH_3$ | H | 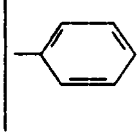 |

Tabelle 2

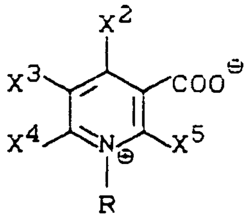

(Ib)

| R | $X^5$ | $X^2$ | $X^3$ | $X^4$ |
|---|---|---|---|---|
| $-C_3H_7$-n | H | H | H | H |
| $-C_4H_9$-n | " | " | " | " |
| $-C_5H_{11}$-n | " | " | " | " |
| $-C_6H_{13}$-n | " | " | " | " |
| $-C_3H_7$-iso | " | " | " | " |
| $-C_4H_9$-iso | " | " | " | " |
| $-C_4H_9$-tert. | " | " | " | " |
| $CF_3$ | " | " | " | " |
| $-CH_2-Si(CH_3)_3$ | " | " | " | " |
| $-CH_2-CH=CH_2$ | " | " | " | " |
| $-CH_2-CH=CH-$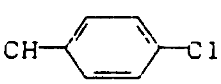 | H | H | H | H |
| $-CH_2-C\equiv CH$ | " | " | " | " |
| $-CH_2-C(=O)-CH_3$ | " | " | " | " |

Tabelle 2 (Fortsetzung)

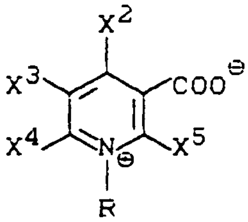

(Ib)

| R | $X^5$ | $X^2$ | $X^3$ | $X^4$ |
|---|---|---|---|---|
| $-CH_2-\underset{O}{\underset{\|}{C}}-C(CH_3)_3$ | H | H | H | H |
| 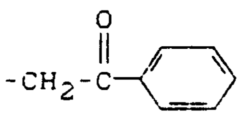 | " | " | " | " |
| $-CH_2-COOH$ | " | " | " | " |
| $-CH_2-COOCH_3$ | " | " | " | " |

Tabelle 2 (Fortsetzung)

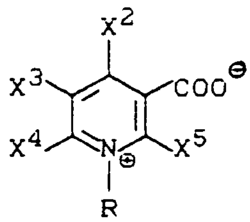

(Ib)

| R | $X^5$ | $X^2$ | $X^3$ | $X^4$ |
|---|---|---|---|---|
| $-CH_2-COO-CH_2-$ 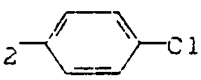 | H | H | H | H |
| $-CH_2-CH_2-COO-C_2H_5$ | " | " | " | " |
| $-CH_2-CH_2-COO-$ 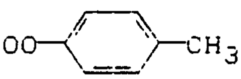 | " | " | " | " |
| $-CH_2-CO-NH_2$ | " | " | " | " |
| $-CH_2-CO-NH-CH_3$ | " | " | " | " |
| $-CH_2-CH=CH-$ 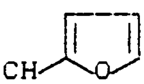 | " | " | " | " |

Tabelle 2 (Fortsetzung)

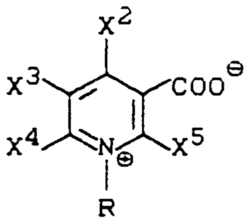

(Ib)

| R | $X^5$ | $X^2$ | $X^3$ | $X^4$ |
|---|---|---|---|---|
| $-CH_2-CH_2-S-CH_3$ | H | H | H | H |
| $-CH_2-CO-NH-$ 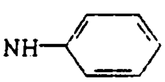 | " | " | " | " |
| $-CH_2-CO-NH-CH_2-COO-CH_3$ | " | " | " | " |
| $-CH_2-CH_2-OH$ | " | " | " | " |
| $-CH_2-CH_2-O-C(=O)-CH_3$ | " | " | " | " |
| $-CH_2-CH_2-O-C(=O)-C_{10}H_{21}$ | " | " | " | " |
| $-CH_2-CH_2-COO-C_{12}H_{25}$ | " | " | " | " |

Tabelle 2 (Fortsetzung)

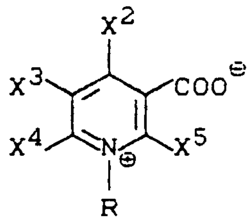

(Ib)

| R | $X^5$ | $X^2$ | $X^3$ | $X^4$ |
|---|---|---|---|---|
| 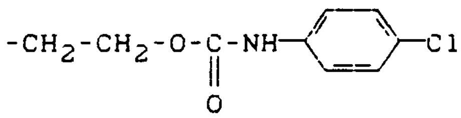 | H | H | H | H |
| $-CH_2-CH_2-O-CH_3$ | " | " | " | " |
| 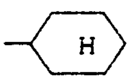 | " | " | " | " |
| 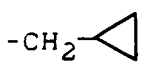 | " | " | " | " |
| 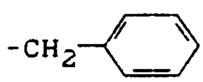 | " | " | " | " |
| 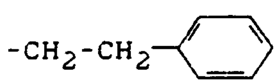 | " | " | " | " |

Tabelle 2 (Fortsetzung)

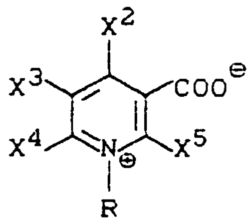

(Ib)

| R | $X^5$ | $X^2$ | $X^3$ | $X^4$ |
|---|---|---|---|---|
| 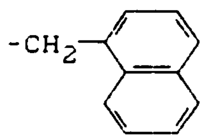 | H | H | H | H |
| 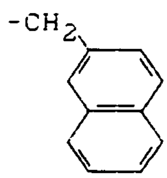 | " | " | " | " |
| 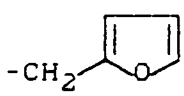 | " | " | " | " |
| 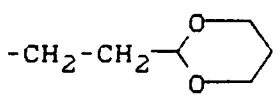 | " | " | " | " |

Tabelle 2 (Fortsetzung)

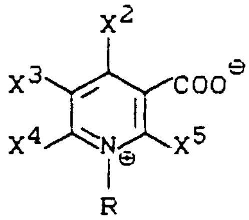

(Ib)

| R | $X^5$ | $X^2$ | $X^3$ | $X^4$ |
| --- | --- | --- | --- | --- |
| 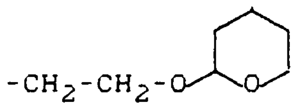 | H | H | H | H |
| 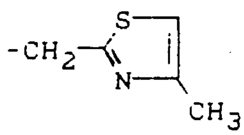 | " | " | " | " |
| 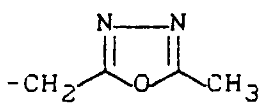 | " | " | " | " |
| 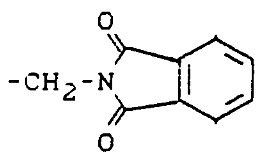 | " | " | " | " |
| 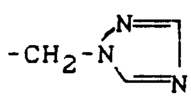 | " | " | " | " |
| 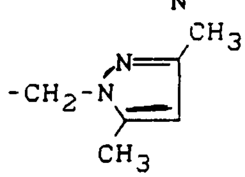 | H | H | H | H |

Tabelle 2 (Fortsetzung)

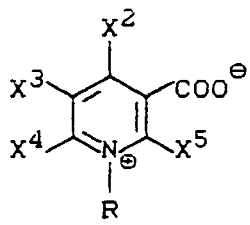

(Ib)

| R | $X^5$ | $X^2$ | $X^3$ | $X^4$ |
|---|---|---|---|---|
| $-CH_3$ | $CH_3$ | H | H | H |
| $-CH_3$ | H | $CH_3$ | H | H |
| $-CH_3$ | H | H | $CH_3$ | H |
| $-CH_3$ | H | H | H | $CH_3$ |
| $-CH_3$ | $CH_3$ | $CH_3$ | H | H |
| $-CH_3$ | $CH_3$ | H | $CH_3$ | H |
| $-CH_3$ | $CH_3$ | H | H | $CH_3$ |
| $-CH_3$ | H | $CH_3$ | $CH_3$ | H |
| $-CH_3$ | H | $CH_3$ | H | $CH_3$ |

Tabelle 2 (Fortsetzung)

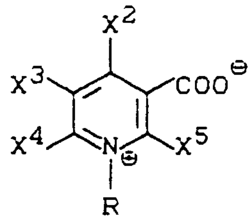

(Ib)

| R | $X^5$ | $X^2$ | $X^3$ | $X^4$ |
|---|---|---|---|---|
| $-CH_3$ | H | H | $CH_3$ | $CH_3$ |
| $-CH_3$ | $CH_3$ | $CH_3$ | H | $CH_3$ |
| $-CH_3$ | $CH_3$ | $CH_3$ | $CH_3$ | $CH_3$ |
| $-CH_3$ | $C_2H_5$ | H | H | H |
| $-CH_3$ | H | H | H | $C_2H_5$ |
| $-CH_3$ | H | H | H | $C_3H_7$-n |

Tabelle 2 (Fortsetzung)

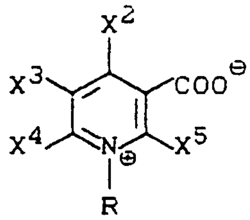

(Ib)

| R | $X^5$ | $X^2$ | $X^3$ | $X^4$ |
|---|---|---|---|---|
| $-CH_3$ | Cl | H | H | H |
| $-CH_3$ | H | Cl | H | H |
| $-CH_3$ | H | H | Cl | H |
| $-CH_3$ | H | H | H | Cl |
| $-CH_3$ | Cl | Cl | H | H |
| $-CH_3$ | Cl | H | Cl | H |
| $-CH_3$ | H | Cl | H | Cl |
| $-CH_3$ | H | H | Cl | Cl |
| $-CH_3$ | Br | H | H | H |
| $-CH_3$ | H | H | Br | H |
| $-CH_3$ | F | H | H | H |
| $-CH_3$ | H | H | H | F |
| $-CH_3$ | H | H | F | H |
| $-CH_3$ | $CH_3$ | $CH_3$ | Cl | $CH_3$ |
| $-CH_3$ | $CH_3$ | $CF_3$ | H | $CF_3$ |
| $-CH_3$ | H | H | H | $CF_3$ |

Tabelle 2 (Fortsetzung)

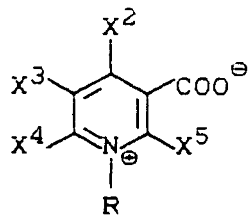

(Ib)

| R | $X^5$ | $X^2$ | $X^3$ | $X^4$ |
|---|---|---|---|---|
| $-CH_3$ | $CF_3$ | $CH_3$ | $CH_3$ | $CH_3$ |
| $-CH_3$ | $CH_3$ | $C_2F_5$ | H | $C_2F_5$ |
| $-CH_3$ | $OCF_3$ | $CH_3$ | H | $CH_3$ |
| $-CH_3$ | $CH_3$ | $OCF_3$ | H | $OCF_3$ |
| $-CH_3$ | $SCF_3$ | H | H | H |
| $-CH_3$ | H | H | OH | H |
| $-CH_3$ | H | OH | H | H |
| $-CH_3$ | OH | H | H | H |
| $-CH_3$ | H | SH | H | H |
| $-CH_3$ | H | H | SH | H |
| $-CH_3$ | SH | H | H | H |
| $-CH_3$ | $NH_2$ | H | H | H |
| $-CH_3$ | H | H | $NH_2$ | H |
| $-CH_3$ | H | H | $OCH_3$ | H |
| $-CH_3$ | H | $OCH_3$ | H | H |
| $-CH_3$ | $OCH_3$ | H | H | H |
| $-CH_3$ | $OC_2H_5$ | H | H | H |
| $-CH_3$ | H | $SCH_3$ | H | H |
| $-CH_3$ | H | H | $SCH_3$ | H |

Tabelle 2 (Fortsetzung)

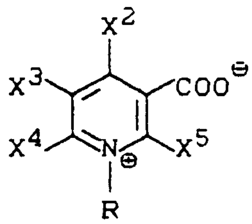

(Ib)

| R | $X^5$ | $X^2$ | $X^3$ | $X^4$ |
|---|---|---|---|---|
| $-CH_3$ | $SC_2H_5$ | H | H | H |
| $-CH_3$ | $NH-CH_3$ | H | H | H |
| $-CH_3$ | $N(CH_3)_2$ | H | H | H |
| $-CH_3$ | H | H | $N(C_2H_5)_2$ | H |
| $-CH_3$ | H | H | H | NH 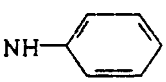 |
| $-CH_3$ | $NH-CH_2$ 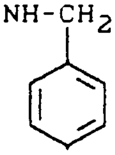 | H | H | H |
| $-CH_3$ | H | 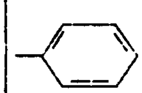 | H | 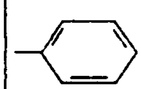 |
| $-CH_3$ | 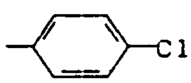 | $CH_3$ | H | $CH_3$ |

Tabelle 2 (Fortsetzung)

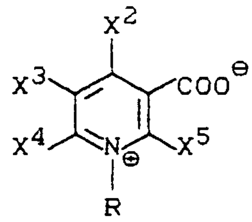

(Ib)

| R | $X^5$ | $X^2$ | $X^3$ | $X^4$ |
|---|---|---|---|---|
| $-CH_3$ | 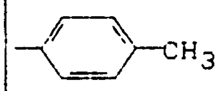 | H | H | 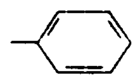 |
| $-CH_3$ | 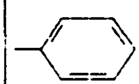 | $CH_3$ | H | 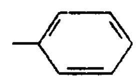 |
| $-C_2H_5$ | $CH_3$ | H | H | H |
| $-C_2H_5$ | H | $CH_3$ | H | H |
| $-C_2H5$ | H | H | $CH_3$ | H |
| $-C_2H_5$ | H | H | H | $CH_3$ |
| $-C_2H_5$ | $CH_3$ | $CH_3$ | H | H |
| $-C_2H_5$ | $CH_3$ | H | $CH_3$ | H |
| $-C_2H_5$ | $CH_3$ | H | H | $CH_3$ |

Tabelle 2 (Fortsetzung)

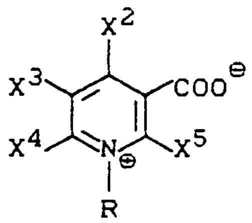

(Ib)

| R | $X^5$ | $X^2$ | $X^3$ | $X^4$ |
|---|---|---|---|---|
| $-C_2H_5$ | H | $CH_3$ | $CH_3$ | H |
| $-C_2H_5$ | H | $CH_3$ | H | $CH_3$ |
| $-C_2H_5$ | H | H | $CH_3$ | $CH_3$ |
| $-C_2H_5$ | $CH_3$ | $CH_3$ | H | $CH_3$ |
| $-C_2H_5$ | $CH_3$ | $CH_3$ | $CH_3$ | $CH_3$ |
| $-C_2H_5$ | $C_2H_5$ | H | H | H |
| $-C_2H_5$ | H | H | H | $C_2H_5$ |
| $-C_2H_5$ | H | H | H | $C_3H_7$-n |
| $-C_2H_5$ | Cl | H | H | H |

Tabelle 2 (Fortsetzung)

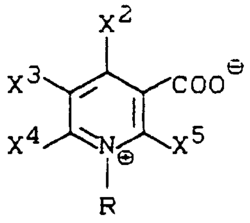

(Ib)

| R | $X^5$ | $X^2$ | $X^3$ | $X^4$ |
|---|---|---|---|---|
| $-C_2H_5$ | H | Cl | H | H |
| $-C_2H_5$ | H | H | Cl | H |
| $-C_2H_5$ | H | H | H | Cl |
| $-C_2H_5$ | Cl | Cl | H | H |
| $-C_2H_5$ | Cl | H | Cl | H |
| $-C_2H_5$ | H | Cl | H | Cl |
| $-C_2H_5$ | H | H | Cl | Cl |
| $-C_2H_5$ | Br | H | H | H |
| $-C_2H_5$ | H | H | Br | H |
| $-C_2H_5$ | F | H | H | H |

Tabelle 2 (Fortsetzung)

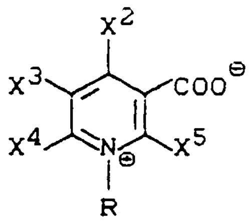

(Ib)

| R | $X^5$ | $X^2$ | $X^3$ | $X^4$ |
|---|---|---|---|---|
| $-C_2H_5$ | H | H | H | F |
| $-C_2H_5$ | H | H | F | H |
| $-C_2H_5$ | $CH_3$ | $CH_3$ | Cl | $CH_3$ |
| $-C_2H_5$ | $CH_3$ | $CF_3$ | H | $CF_3$ |
| $-C_2H_5$ | H | H | H | $CF_3$ |
| $-C_2H_5$ | $CF_3$ | $CH_3$ | $CH_3$ | $CH_3$ |
| $-C_2H_5$ | $CH_3$ | $C_2F_5$ | H | $C_2F_5$ |
| $-C_2H_5$ | $OCF_3$ | $CH_3$ | H | $CH_3$ |
| $-C_2H_5$ | $CH_3$ | $OCF_3$ | H | $OCF_3$ |
| $-C_2H_5$ | $SCF_3$ | H | H | H |
| $-C_2H_5$ | H | H | OH | H |
| $-C_2H_5$ | H | OH | H | H |
| $-C_2H_5$ | OH | H | H | H |
| $-C_2H_5$ | H | SH | H | H |
| $-C_2H_5$ | H | H | SH | H |
| $-C_2H_5$ | SH | H | H | H |
| $-C_2H_5$ | $NH_2$ | H | H | H |
| $-C_2H_5$ | H | H | $NH_2$ | H |
| $-C_2H_5$ | H | H | $OCH_3$ | H |
| $-C_2H_5$ | H | $OCH_3$ | H | H |

Tabelle 2 (Fortsetzung)

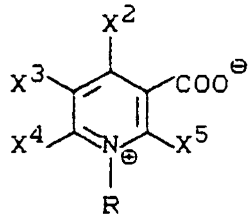

(Ib)

| R | $X^5$ | $X^2$ | $X^3$ | $X^4$ |
|---|---|---|---|---|
| $-C_2H_5$ | $OCH_3$ | H | H | H |
| $-C_2H_5$ | $OC_2H_5$ | H | H | H |
| $-C_2H_5$ | H | $SCH_3$ | H | H |
| $-C_2H_5$ | H | H | $SCH_3$ | H |
| $-C_2H_5$ | $SC_2H_5$ | H | H | H |
| $-C_2H_5$ | $NH-CH_3$ | H | H | H |
| $-C_2H_5$ | $N(CH_3)_2$ | H | H | H |
| $-C_2H_5$ | H | H | $N(C_2H_5)_2$ | H |
| $-C_2H_5$ | H | H | H | NH 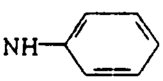 |

Tabelle 2 (Fortsetzung)

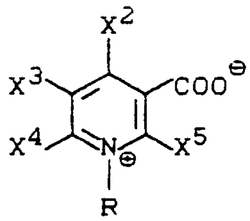

(Ib)

| R | $X^5$ | $X^2$ | $X^3$ | $X^4$ |
|---|---|---|---|---|
| $-C_2H_5$ | $-NH-CH_2$ 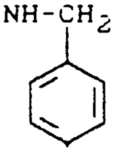 | H | H | H |
| $-C_2H_5$ | H | 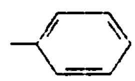 | H | 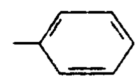 |
| $-C_2H_5$ | 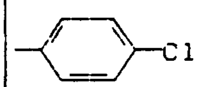 | $CH_3$ | H | $CH_3$ |
| $-C_2H_5$ | 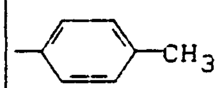 | H | H | 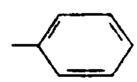 |
| $-C_2H_5$ | 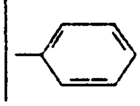 | $CH_3$ | H | 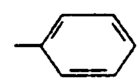 |

Tabelle 2 (Fortsetzung)

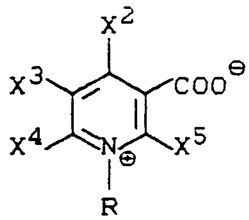

(Ib)

| R | $X^5$ | $X^2$ | $X^3$ | $X^4$ |
|---|---|---|---|---|
| $-C_3H_7-n$ | $CH_3$ | H | H | H |
| $-C_4H_9-n$ | H | $CH_3$ | H | H |
| $-CH_2-CH=CH_2$ | H | H | $CH_3$ | H |
| $-CH_2-COO-C_2H_5$ | H | H | H | $CH_3$ |
| $-CH_2-CO-$ 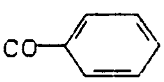 | $CH_3$ | $CH_3$ | H | H |
| $-CH_2-CH_2-OH$ | $CH_3$ | H | $CH_3$ | H |
| $-CH_2-CH_2-O-C(=O)-CH_3$ | $CH_3$ | H | H | $CH_3$ |

Tabelle 2 (Fortsetzung)

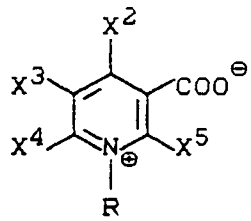

(Ib)

| R | $X^5$ | $X^2$ | $X^3$ | $X^4$ |
|---|---|---|---|---|
| $-CH_2-$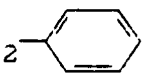 | H | $CH_3$ | $CH_3$ | H |
| $-CH_2-$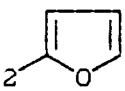 | H | $CH_3$ | H | $CH_3$ |
| $-CH_2-O-C_2H_5$ | H | H | $CH_3$ | $CH_3$ |
| $-CH_2-$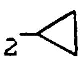 | $CH_3$ | $CH_3$ | H | $CH_3$ |
| $-CH_2-S-$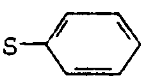 | $CH_3$ | $CH_3$ | $CH_3$ | $CH_3$ |

Tabelle 2 (Fortsetzung)

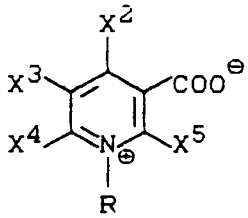

(Ib)

| R | $X^5$ | $X^2$ | $X^3$ | $X^4$ |
|---|---|---|---|---|
| $-CH_2-CH_2-C(=O)-NH-CH_3$ | $C_2H_5$ | H | H | H |
| $-CH_2-C\equiv CH$ | H | H | H | $C_2H_5$ |
| $-CH_2-$ 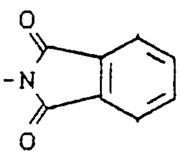 | H | H | H | $C_3H_7$-n |
| $-CH_2-$ 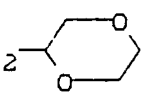 | Cl | H | H | H |
| $-CH_2-CH_2-$ 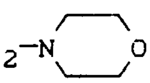 | H | Cl | H | H |
| $-CH_2-CH_2-CH(OC_2H_5)_2$ | H | H | Cl | H |
| $CF_3$ | H | H | H | Cl |

Tabelle 2 (Fortsetzung)

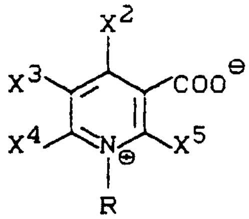

(Ib)

| R | $X^5$ | $X^2$ | $X^3$ | $X^4$ |
|---|---|---|---|---|
| 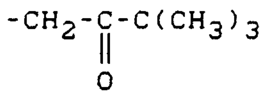 | Cl | Cl | H | H |
| $-CH_2-CH_2-S-CH_3$ | Cl | H | Cl | H |
| $-C_3H_7$-n | H | Cl | H | Cl |
| $-C_6H_{13}$-n | H | H | Cl | Cl |
| $-CH_2-CH_2-O-CH_3$ | Br | H | H | H |
| 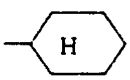 | H | H | Br | H |
| 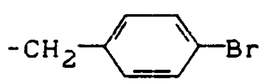 | F | H | H | H |
| $-C_3H_7$-n | H | H | F | H |

Tabelle 2 (Fortsetzung)

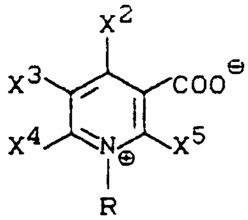

(Ib)

| R | $X^5$ | $X^2$ | $X^3$ | $X^4$ |
|---|---|---|---|---|
| $-CH_2-CH_2-$ 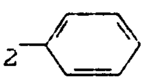 | H | H | H | F |
| $-C_4H_9$-iso | $CH_3$ | $CH_3$ | Cl | $CH_3$ |
| $-CH_2-$ 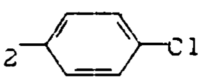 | $CH_3$ | $CF_3$ | H | $CF_3$ |
| $-CH_2-CO-CH_3$ | H | H | H | $CF_3$ |
| 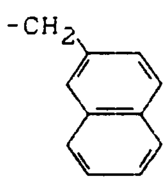 | $CF_3$ | $CH_3$ | $CH_3$ | $CH_3$ |
| $-CH_2-$ 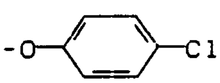 | $CH_3$ | $C_2F_5$ | H | $C_2F_5$ |

Tabelle 2 (Fortsetzung)

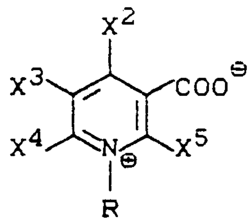

(Ib)

| R | $X^5$ | $X^2$ | $X^3$ | $X^4$ |
|---|---|---|---|---|
| $-C_5H_{11}$-n | $OCF_3$ | $CH_3$ | H | $CH_3$ |
| $-C_3H_7$-n | $CH_3$ | $OCF_3$ | H | $OCF_3$ |
| $-CH_2-$ 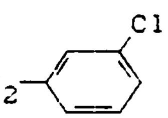 | $SCF_3$ | H | H | H |
| $-CH_2-$ 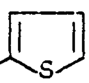 | H | H | OH | H |
| $-CH_2-$ 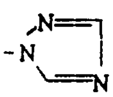 | H | OH | H | H |
| $-C_3H_7$-n | OH | H | H | H |
| $-C(CH_3)_3$ | H | SH | H | H |

Tabelle 2 (Fortsetzung)

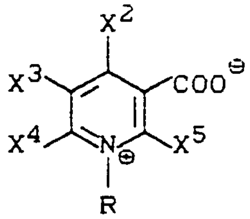

(Ib)

| R | $X^5$ | $X^2$ | $X^3$ | $X^4$ |
|---|---|---|---|---|
| $-CH_2$ 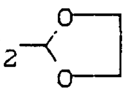 | H | H | SH | H |
| $-CH_2-CH_2-OH$ | SH | H | H | H |
| $-C_3H_7-n$ | $NH_2$ | H | H | H |
| $-C_4H_9-n$ | H | H | $NH_2$ | H |
| $-CH_2$ 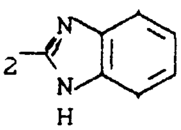 | H | H | $OCH_3$ | H |
| $-CH_2-CH_2-$ 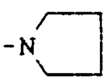 | H | $OCH_3$ | H | H |

Tabelle 2 (Fortsetzung)

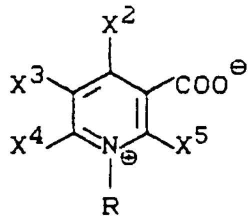

(Ib)

| R | $X^5$ | $X^2$ | $X^3$ | $X^4$ |
|---|---|---|---|---|
| $-CH_2-CN$ | $OCH_3$ | H | H | H |
| $-CH_2-CH_2-S-CH_3$ | $OC_2H_5$ | H | H | H |
| $-CH_2-CH=CH_2$ | H | $SCH_3$ | H | H |
| $-CH_2-CF_3$ | H | H | $SCH_3$ | H |
| $-CH_2-CH_2-C(=O)-NH-CH_3$ | $SC_2H_5$ | H | H | H |
| $-C_5H_{11}-n$ | $NH-CH_3$ | H | H | H |
| $-C_3H_7-n$ | $N(CH_3)_2$ | H | H | H |

Tabelle 2 (Fortsetzung)

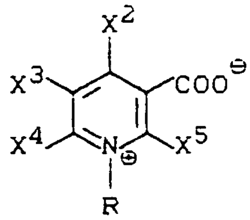

(Ib)

| R | $X^5$ | $X^2$ | $X^3$ | $X^4$ |
|---|---|---|---|---|
| $-CH_2-CH_2-CO-CH_3$ | H | H | $N(C_2H_5)_2$ | H |
| $-CH_2-CH_2-CO-NH_2$ | H | H | H | NH- 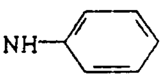 |
| $-CH_2-$ 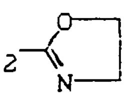 | $NH-CH_2$ 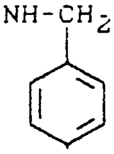 | H | H | H |
| $-C_3H_7-n$ | H | 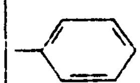 | H | 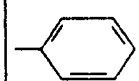 |
| $-C_4H_9-n$ | 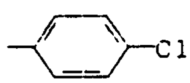 | $CH_3$ | H | $CH_3$ |

Tabelle 2 (Fortsetzung)

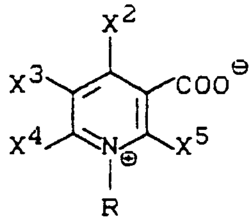

(Ib)

| R | $X^5$ | $X^2$ | $X^3$ | $X^4$ |
|---|---|---|---|---|
| $-CH_2-CH_2-O-CH_3$ | 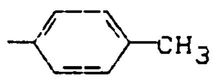 | H | H | 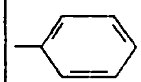 |
| $-C_6H_{13}-n$ | 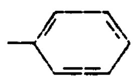 | $CH_3$ | H | 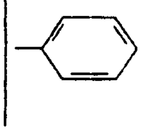 |

Tabelle 3

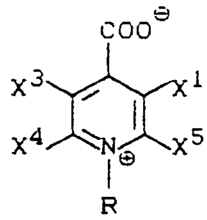

(Ic)

| R | $X^5$ | $X^1$ | $X^3$ | $X^4$ |
|---|---|---|---|---|
| $-C_3H_7$-n | H | H | H | H |
| $-C_4H_9$-n | " | " | " | " |
| $-C_5H_{11}$-n | " | " | " | " |
| $-C_6H_{13}$-n | " | " | " | " |
| $-C_3H_7$-iso | " | " | " | " |
| $-C_4H_9$-iso | " | " | " | " |
| $-CH_2-CF_3$ | " | " | " | " |
| $-CH_2-CH_2-Cl$ | " | " | " | " |
| $-CH_2-CH=CH-CH_3$ | " | " | " | " |
| $-CH_2-CH=CH_2$ | " | " | " | " |
| 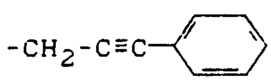 | H | H | H | H |
| 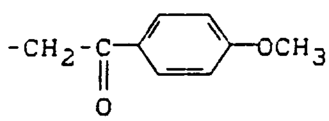 | " | " | " | " |
| $-CH_2-CH_2-SO-CH_3$ | " | " | " | " |
| $-CH_2-CO-O-C_2H_5$ | " | " | " | " |
| 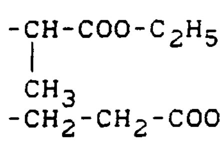 | " | " | " | " |
| $-CH_2-CH_2-COO-C_3H_7$ | " | " | " | " |
| 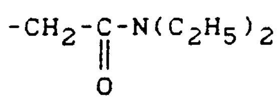 | " | " | " | " |
| 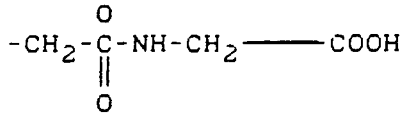 | " | " | " | " |

Tabelle 3 (Fortsetzung)

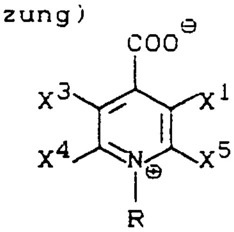

(Ic)

| R | $X^5$ | $X^1$ | $X^3$ | $X^4$ |
|---|---|---|---|---|
| 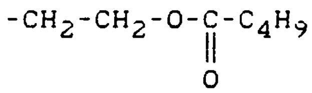 | H | H | H | H |
| 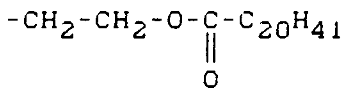 | H | H | H | H |
| 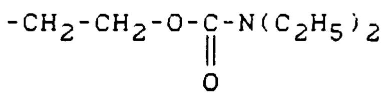 | " | " | " | " |
| $-CH_2-O-C_2H_5$ | " | " | " | " |
| 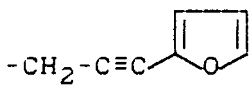 | " | " | " | " |
| 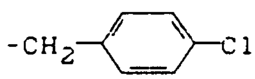 | H | H | H | H |
| 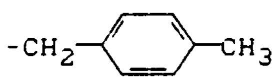 | " | " | " | " |
| 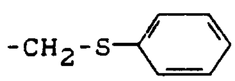 | " | " | " | " |
| 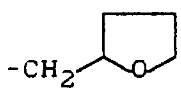 | " | " | " | " |
| 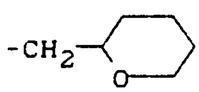 | " | " | " | " |
| 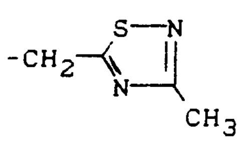 | " | " | " | " |

Tabelle 3 (Fortsetzung)

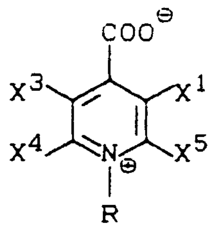

(Ic)

| R | $X^5$ | $X^1$ | $X^3$ | $X^4$ |
| --- | --- | --- | --- | --- |
| 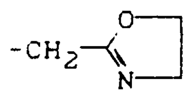 | H | H | H | H |
| 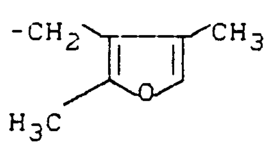 | " | " | " | " |
| 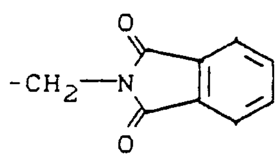 | " | " | " | " |
| 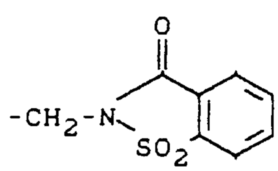 | " | " | " | " |

Tabelle 3 (Fortsetzung)

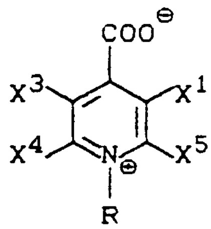

(Ic)

| R | $X^5$ | $X^1$ | $X^3$ | $X^4$ |
|---|---|---|---|---|
| $-CH_2-CH_2-$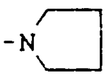 | H | H | H | H |
| $-CH_2-COO-C_{10}H_{21}$ | " | " | " | " |
| 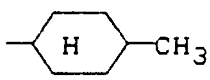 | " | " | " | " |
| $-CH_2$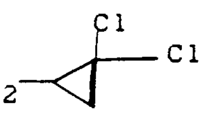 | " | " | " | " |
| 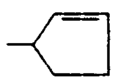 | " | " | " | " |
| $-CH_2$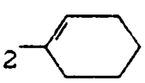 | " | " | " | " |

Tabelle 3 (Fortsetzung)

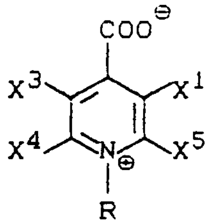

(Ic)

| R | $X^5$ | $X^1$ | $X^3$ | $X^4$ |
|---|---|---|---|---|
| $-CH(CH_3)-CO-CH_3$ | H | H | H | H |
| $-CH_2-CH_2-CH_2-COO-CH_3$ | " | " | " | " |
| $-CH_2-CO-C_2H_5$ | " | " | " | " |
| $-CH_2-$ 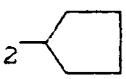 | " | " | " | " |
| $-CH_2-CO-NH-CH_2-CO-NH$ 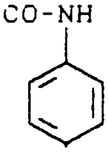 | " | " | " | " |

Tabelle 3 (Fortsetzung)

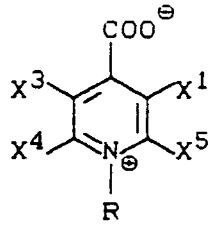

(Ic)

| R | $X^5$ | $X^1$ | $X^3$ | $X^4$ |
|---|---|---|---|---|
| $-CH_2-CH_2-CO-NH-CH_2-CO(OC_2H_5)$ | H | H | H | H |
| $-CH_3$ | $CH_3$ | H | H | H |
| $-CH_3$ | H | $CH_3$ | H | H |
| $-CH_3$ | H | H | $CH_3$ | H |
| $-CH_3$ | H | H | H | $CH_3$ |
| $-CH_3$ | $CH_3$ | $CH_3$ | H | H |
| $-CH_3$ | $CH_3$ | H | $CH_3$ | H |
| $-CH_3$ | $CH_3$ | H | H | $CH_3$ |
| $-CH_3$ | H | $CH_3$ | $CH_3$ | H |
| $-CH_3$ | H | $CH_3$ | H | $CH_3$ |

Tabelle 3 (Fortsetzung)

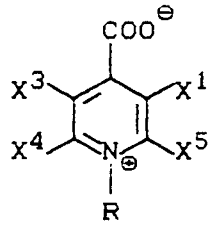

(Ic)

| R | $X^5$ | $X^1$ | $X^3$ | $X^4$ |
|---|---|---|---|---|
| $-CH_3$ | H | H | $CH_3$ | $CH_3$ |
| $-CH_3$ | $CH_3$ | $CH_3$ | H | $CH_3$ |
| $-CH_3$ | $CH_3$ | $CH_3$ | $CH_3$ | $CH_3$ |
| $-CH_3$ | $C_2H_5$ | H | H | H |
| $-CH_3$ | H | H | H | $C_2H_5$ |
| $-CH_3$ | H | H | H | $C_3H_7$-n |
| $-CH_3$ | Cl | H | H | H |
| $-CH_3$ | H | Cl | H | H |
| $-CH_3$ | H | H | Cl | H |
| $-CH_3$ | H | H | H | Cl |
| $-CH_3$ | Cl | Cl | H | H |
| $-CH_3$ | Cl | H | Cl | H |

Tabelle 3 (Fortsetzung)

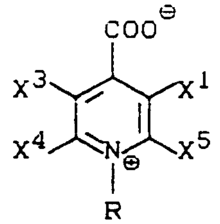

(Ic)

| R | $X^5$ | $X^1$ | $X^3$ | $X^4$ |
|---|---|---|---|---|
| $-CH_3$ | H | Cl | H | Cl |
| $-CH_3$ | H | H | Cl | Cl |
| $-CH_3$ | Br | H | H | H |
| $-CH_3$ | H | H | Br | H |
| $-CH_3$ | F | H | H | H |
| $-CH_3$ | H | H | H | F |
| $-CH_3$ | H | H | F | H |
| $-CH_3$ | $CH_3$ | $CH_3$ | Cl | $CH_3$ |
| $-CH_3$ | $CH_3$ | $CF_3$ | H | $CF_3$ |
| $-CH_3$ | H | H | H | $CF_3$ |
| $-CH_3$ | $CF_3$ | $CH_3$ | $CH_3$ | $CH_3$ |
| $-CH_3$ | $CH_3$ | $C_2F_5$ | H | $C_2F_5$ |
| $-CH_3$ | $OCF_3$ | $CH_3$ | H | $CH_3$ |
| $-CH_3$ | $CH_3$ | $OCF_3$ | H | $OCF_3$ |
| $-CH_3$ | $SCF_3$ | H | H | H |
| $-CH_3$ | H | H | OH | H |
| $-CH_3$ | H | OH | H | H |
| $-CH_3$ | OH | H | H | H |
| $-CH_3$ | H | SH | H | H |

Tabelle 3 (Fortsetzung)

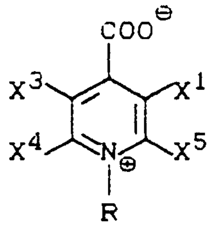

(Ic)

| R | $X^5$ | $X^1$ | $X^3$ | $X^4$ |
|---|---|---|---|---|
| $-CH_3$ | H | H | SH | H |
| $-CH_3$ | SH | H | H | H |
| $-CH_3$ | $NH_2$ | H | H | H |
| $-CH_3$ | H | H | $NH_2$ | H |
| $-CH_3$ | H | H | $OCH_3$ | H |
| $-CH_3$ | H | $OCH_3$ | H | H |
| $-CH_3$ | $OCH_3$ | H | H | H |
| $-CH_3$ | $OC_2H_5$ | H | H | H |
| $-CH_3$ | H | $SCH_3$ | H | H |
| $-CH_3$ | H | H | $SCH_3$ | H |

Tabelle 3 (Fortsetzung)

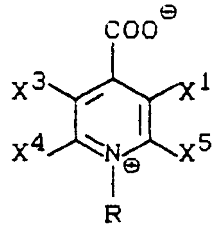

(Ic)

| R | $X^5$ | $X^1$ | $X^3$ | $X^4$ |
|---|---|---|---|---|
| $-CH_3$ | $SC_2H_5$ | H | H | H |
| $-CH_3$ | $NH-CH_3$ | H | H | H |
| $-CH_3$ | $N(CH_3)_2$ | H | H | H |
| $-CH_3$ | H | H | $N(C_2H_5)_2$ | H |
| $-CH_3$ | H | H | H | 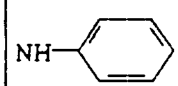 |
| $-CH_3$ | $NH-CH_2$ 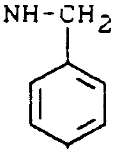 | H | H | H |
| $-CH_3$ | H | 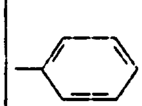 | H | 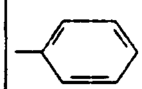 |
| $-CH_3$ | 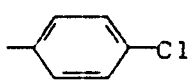 | $CH_3$ | H | $CH_3$ |

Tabelle 3 (Fortsetzung)

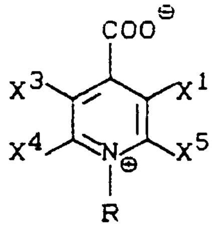

(Ic)

| R | $X^5$ | $X^1$ | $X^3$ | $X^4$ |
|---|---|---|---|---|
| $-CH_3$ | 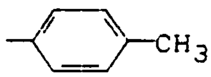 | H | H | 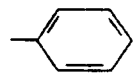 |
| $-CH_3$ | 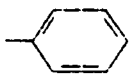 | $CH_3$ | H | 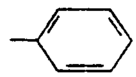 |
| $-C_2H_5$ | $CH_3$ | H | H | H |
| $-C_2H_5$ | H | $CH_3$ | H | H |
| $-C_2H5$ | H | H | $CH_3$ | H |
| $-C_2H_5$ | H | H | H | $CH_3$ |
| $-C_2H_5$ | $CH_3$ | $CH_3$ | H | H |
| $-C_2H_5$ | $CH_3$ | H | $CH_3$ | H |
| $-C_2H_5$ | $CH_3$ | H | H | $CH_3$ |

Tabelle 3 (Fortsetzung)

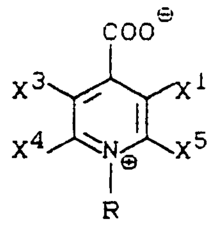

(Ic)

| R | $X^5$ | $X^1$ | $X^3$ | $X^4$ |
|---|---|---|---|---|
| $-C_2H_5$ | H | $CH_3$ | $CH_3$ | H |
| $-C_2H_5$ | H | $CH_3$ | H | $CH_3$ |
| $-C_2H_5$ | H | H | $CH_3$ | $CH_3$ |
| $-C_2H_5$ | $CH_3$ | $CH_3$ | H | $CH_3$ |
| $-C_2H_5$ | $CH_3$ | $CH_3$ | $CH_3$ | $CH_3$ |
| $-C_2H_5$ | $C_2H_5$ | H | H | H |
| $-C_2H_5$ | H | H | H | $C_2H_5$ |
| $-C_2H_5$ | H | H | H | $C_3H_7$-n |
| $-C_2H_5$ | Cl | H | H | H |
| $-C_2H_5$ | H | Cl | H | H |
| $-C_2H_5$ | H | H | Cl | H |
| $-C_2H_5$ | H | H | H | Cl |
| $-C_2H_5$ | Cl | Cl | H | H |
| $-C_2H_5$ | Cl | H | Cl | H |
| $-C_2H_5$ | H | Cl | H | Cl |
| $-C_2H_5$ | H | H | Cl | Cl |
| $-C_2H_5$ | Br | H | H | H |
| $-C_2H_5$ | H | H | Br | H |
| $-C_2H_5$ | F | H | H | H |

Tabelle 3 (Fortsetzung)

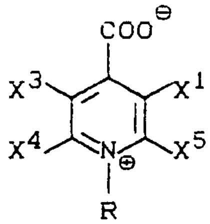

(Ic)

| R | $X^5$ | $X^1$ | $X^3$ | $X^4$ |
|---|---|---|---|---|
| $-C_2H_5$ | H | H | H | F |
| $-C_2H_5$ | H | H | F | H |
| $-C_2H_5$ | $CH_3$ | $CH_3$ | Cl | $CH_3$ |
| $-C_2H_5$ | $CH_3$ | $CF_3$ | H | $CF_3$ |
| $-C_2H_5$ | H | H | H | $CF_3$ |
| $-C_2H_5$ | $CF_3$ | $CH_3$ | $CH_3$ | $CH_3$ |
| $-C_2H_5$ | $CH_3$ | $C_2F_5$ | H | $C_2F_5$ |
| $-C_2H_5$ | $OCF_3$ | $CH_3$ | H | $CH_3$ |
| $-C_2H_5$ | $CH_3$ | $OCF_3$ | H | $OCF_3$ |
| $-C_2H_5$ | $SCF_3$ | H | H | H |
| $-C_2H_5$ | H | H | OH | H |
| $-C_2H_5$ | H | OH | H | H |
| $-C_2H_5$ | OH | H | H | H |
| $-C_2H_5$ | H | SH | H | H |
| $-C_2H_5$ | H | H | SH | H |
| $-C_2H_5$ | SH | H | H | H |
| $-C_2H_5$ | $NH_2$ | H | H | H |
| $-C_2H_5$ | H | H | $NH_2$ | H |
| $-C_2H_5$ | H | H | $OCH_3$ | H |
| $-C_2H_5$ | H | $OCH_3$ | H | H |

Tabelle 3 (Fortsetzung)

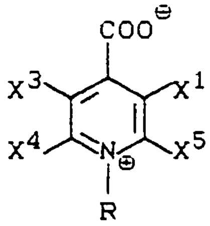

(Ic)

| R | $X^5$ | $X^1$ | $X^3$ | $X^4$ |
|---|---|---|---|---|
| $-C_2H_5$ | $OCH_3$ | H | H | H |
| $-C_2H_5$ | $OC_2H_5$ | H | H | H |
| $-C_2H_5$ | H | $SCH_3$ | H | H |
| $-C_2H_5$ | H | H | $SCH_3$ | H |
| $-C_2H_5$ | $SC_2H_5$ | H | H | H |
| $-C_2H_5$ | $NH-CH_3$ | H | H | H |
| $-C_2H_5$ | $N(CH_3)_2$ | H | H | H |
| $-C_2H_5$ | H | H | $N(C_2H_5)_2$ | H |
| $-C_2H_5$ | H | H | H | NH 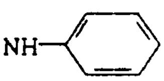 |

Tabelle 3 (Fortsetzung)

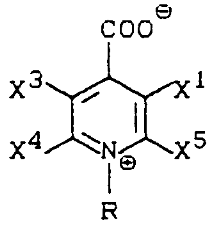

(Ic)

| R | $X^5$ | $X^1$ | $X^3$ | $X^4$ |
|---|---|---|---|---|
| $-C_2H_5$ | $-NH-CH_2$ 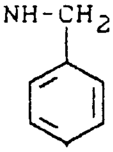 | H | H | H |
| $-C_2H_5$ | H | 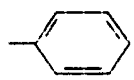 | H | 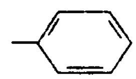 |
| $-C_2H_5$ | 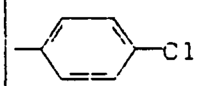 | $CH_3$ | H | $CH_3$ |
| $-C_2H_5$ | 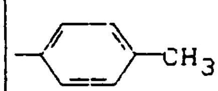 | H | H | 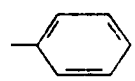 |
| $-C_2H_5$ | 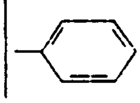 | $CH_3$ | H | 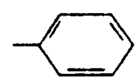 |

Tabelle 3 (Fortsetzung)

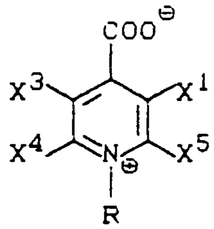

(Ic)

| R | $X^5$ | $X^1$ | $X^3$ | $X^4$ |
|---|---|---|---|---|
| $-C_3H_7-n$ | $CH_3$ | H | H | H |
| $-C_4H_9-n$ | H | $CH_3$ | H | H |
| $-CH_2-CH=CH_2$ | H | H | $CH_3$ | H |
| $-CH_2-COO-C_2H_5$ | H | H | H | $CH_3$ |
| $-CH_2-CO-$ 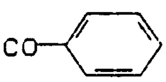 | $CH_3$ | $CH_3$ | H | H |
| $-CH_2-CH_2-OH$ | $CH_3$ | H | $CH_3$ | H |
| $-CH_2-CH_2-O-\underset{\underset{O}{\|}}{C}-CH_3$ | $CH_3$ | H | H | $CH_3$ |

Tabelle 3 (Fortsetzung)

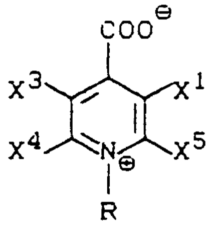

(Ic)

| R | $X^5$ | $X^1$ | $X^3$ | $X^4$ |
|---|---|---|---|---|
| $-CH_2$ 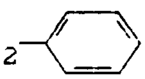 | H | $CH_3$ | $CH_3$ | H |
| $-CH_2$ 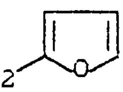 | H | $CH_3$ | H | $CH_3$ |
| $-CH_2-O-C_2H_5$ | H | H | $CH_3$ | $CH_3$ |
| $-CH_2$ 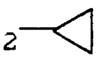 | $CH_3$ | $CH_3$ | H | $CH_3$ |
| $-CH_2-S$ 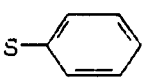 | $CH_3$ | $CH_3$ | $CH_3$ | $CH_3$ |

Tabelle 3 (Fortsetzung)

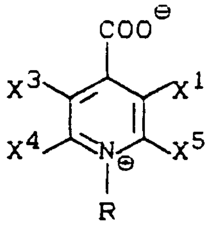

(Ic)

| R | $X^5$ | $X^1$ | $X^3$ | $X^4$ |
|---|---|---|---|---|
| $-CH_2-CH_2-C-NH-CH_3$<br>O | $C_2H_5$ | H | H | H |
| $-CH_2-C \equiv CH$ | H | H | H | $C_2H_5$ |
| $-CH_2-$ 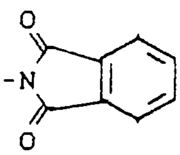 | H | H | H | $C_3H_7$-n |
| $-CH_2-$ 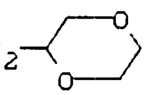 | Cl | H | H | H |
| $-CH_2-CH_2-$ 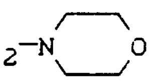 | H | Cl | H | H |
| $-CH_2-CH_2-CH(OC_2H_5)_2$ | H | H | Cl | H |

Tabelle 3 (Fortsetzung)

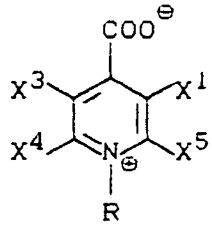

(Ic)

| R | $X^5$ | $X^1$ | $X^3$ | $X^4$ |
|---|---|---|---|---|
| $CF_3$ | H | H | H | Cl |
| $-CH_2-\underset{\underset{O}{\|}}{C}-C(CH_3)_3$ | Cl | Cl | H | H |
| $-CH_2-CH_2-S-CH_3$ | Cl | H | Cl | H |
| $-C_3H_7-n$ | H | Cl | H | Cl |
| $-C_6H_{13}-n$ | H | H | Cl | Cl |
| $-CH_2-CH_2-O-CH_3$ | Br | H | H | H |
| 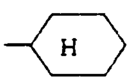 | H | H | Br | H |
| $-CH_2-$ 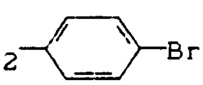 Br | F | H | H | H |
| $-C_3H_7-n$ | H | H | F | H |

Tabelle 3 (Fortsetzung)

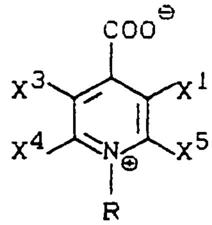

(Ic)

| R | $X^5$ | $X^1$ | $X^3$ | $X^4$ |
|---|---|---|---|---|
| $-CH_2-CH_2-$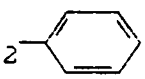 | H | H | H | F |
| $-C_4H_9$-iso | $CH_3$ | $CH_3$ | Cl | $CH_3$ |
| $-CH_2-$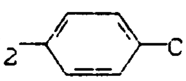$-Cl$ | $CH_3$ | $CF_3$ | H | $CF_3$ |
| $-CH_2-CO-CH_3$ | H | H | H | $CF_3$ |
| $-CH_2$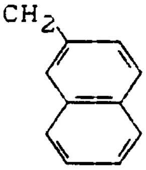 | $CF_3$ | $CH_3$ | $CH_3$ | $CH_3$ |
| $-CH_2-O-$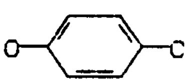$-Cl$ | $CH_3$ | $C_2F_5$ | H | $C_2F_5$ |

Tabelle 3 (Fortsetzung)

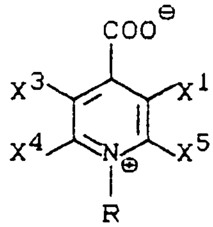

(Ic)

| R | $X^5$ | $X^1$ | $X^3$ | $X^4$ |
|---|---|---|---|---|
| $-C_5H_{11}-n$ | $OCF_3$ | $CH_3$ | H | $CH_3$ |
| $-C_3H_7-n$ | $CH_3$ | $OCF_3$ | H | $OCF_3$ |
| $-CH_2-$ 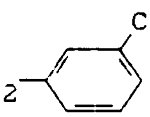 | $SCF_3$ | H | H | H |
| $-CH_2-$ 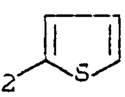 | H | H | OH | H |
| $-CH_2-$ 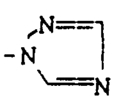 | H | OH | H | H |
| $-C_3H_7-n$ | OH | H | H | H |
| $-C(CH_3)_3$ | H | SH | H | H |

Tabelle 3 (Fortsetzung)

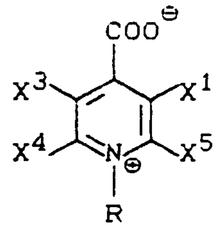

(Ic)

| R | $X^5$ | $X^1$ | $X^3$ | $X^4$ |
|---|---|---|---|---|
| $-CH_2-$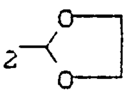 | H | H | SH | H |
| $-CH_2-CH_2-OH$ | SH | H | H | H |
| $-C_3H_7$-n | $NH_2$ | H | H | H |
| $-C_4H_9$-n | H | H | $NH_2$ | H |
| $-CH_2-$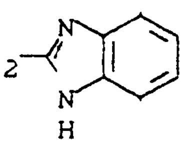 | H | H | $OCH_3$ | H |
| $-CH_2-CH_2-$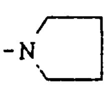 | H | $OCH_3$ | H | H |

Tabelle 3 (Fortsetzung)

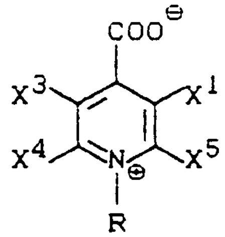

(Ic)

| R | $X^5$ | $X^1$ | $X^3$ | $X^4$ |
|---|---|---|---|---|
| $-CH_2-CN$ | $OCH_3$ | H | H | H |
| $-CH_2-CH_2-S-CH_3$ | $OC_2H_5$ | H | H | H |
| $-CH_2-CH=CH_2$ | H | $SCH_3$ | H | H |
| $-CH_2-CF_3$ | H | H | $SCH_3$ | H |
| $-CH_2-CH_2-C(=O)-NH-CH_3$ | $SC_2H_5$ | H | H | H |
| $-C_5H_{11}-n$ | $NH-CH_3$ | H | H | H |
| $-C_3H_7-n$ | $N(CH_3)_2$ | H | H | H |

Tabelle 3 (Fortsetzung)

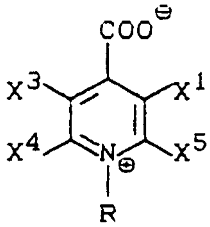

(Ic)

| R | $X^5$ | $X^1$ | $X^3$ | $X^4$ |
|---|---|---|---|---|
| $-CH_2-CH_2-CO-CH_3$ | H | H | $N(C_2H_5)_2$ | H |
| $-CH_2-CH_2-CO-NH_2$ | H | H | H | NH 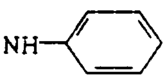 |
| $-CH_2$ 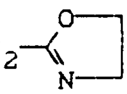 | $NH-CH_2$ 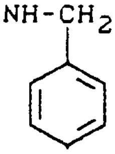 | H | H | H |
| $-C_3H_7-n$ | H | 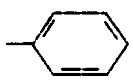 | H | 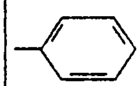 |
| $-C_4H_9-n$ | 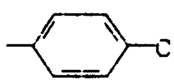 Cl | $CH_3$ | H | $CH_3$ |

Tabelle 3 (Fortsetzung)

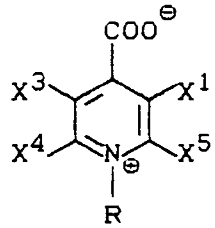 (Ic)

| R | $X^5$ | $X^1$ | $X^3$ | $X^4$ |
|---|---|---|---|---|
| $-CH_2-CH_2-O-CH_3$ | 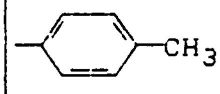 | H | H | 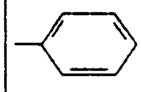 |
| $-C_6H_{13}-n$ | 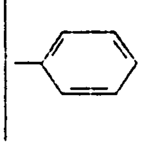 | $CH_3$ | H | 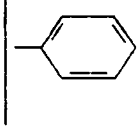 |

Die erfindungsgemäß verwendbaren Pyridinium-Betaine der Formel (I) sind teilweise bekannt (vgl. J. Org. Chem. 26, 1318-1319 (1961), Bull. Soc. Chim. Fr. 1975, 1502, Phytochemistry 23, 1225-1228 (1984) und EP-OS 0 127 670). Sie lassen sich nach bekannten Verfahren herstellen. So erhält man Pyridinium-Betaine der Formel (I), indem man

a) Pyridin-Derivate der Formel

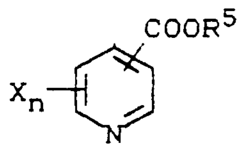 (II)

in welcher

X und n die oben angegebenen Bedeutungen haben und

$R^5$ für Alkyl steht,

mit Verbindungen der Formel

R-Y (III)

in welcher

R die oben angegebene Bedeutung hat und

Y für Chlor, Brom, Iod, Sulfonyl, Alkylsulfat oder den Rest der Formel $O^{\oplus}R^6$ $BF_4^{\ominus}$ steht, worin $R^6$ für Methyl oder Ethyl steht,

in Gegenwart eines Verdünnungsmittels umsetzt und anschließend die so erhaltenen Pyridinium-Salze der Formel

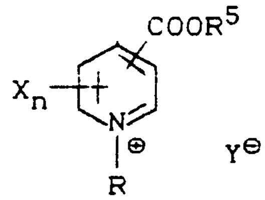

(IV)

in welcher
R, $R^5$, X, Y und n die oben angegebene Bedeutung haben,
entweder

α) an einem basischen Ionenaustauscher in Gegenwart eines Verdünnungsmittels umsetzt oder
β) mit Silberoxid in Gegenwart eines Verdünnungsmittels umsetzt,

oder
b) Pyridincarbonsäuren der Formel

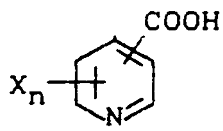

(V)

in welcher

X und n — die oben angegebenen Bedeutungen haben, mit aktivierten Olefinen der Formel

$Z^1 - CH = CH_2$ (VI)

in welcher

$Z^1$ — für einen elektronenziehenden Rest der Formel $-COOR^7$, $-CO-NR^8R^9$, -CN, $-CO-R^{10}$ oder $-SO_2R^{11}$ steht, worin

$R^7$, $R^8$, $R^9$ und $R^{10}$ — unabhängig voneinander für Wasserstoff, Alkyl, Aryl, Aralkyl oder Cycloalkyl stehen und

$R^{11}$ — für Alkyl, Aryl, Aralkyl oder Cycloalkyl steht,

in Gegenwart eines Verdünnungsmittels umsetzt,
oder
c) Pyridin-Derivate der Formel

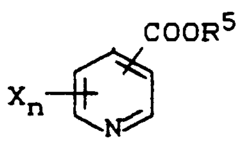

(II)

in welcher

$R^5$, X und n — die oben angegebenen Bedeutungen haben,

gegebenenfalls in Gegenwart eines Verdünnungsmittels thermisch umlagert.

Die bei der Durchführung der obigen Verfahren a) und c) zur Herstellung von Pyridinium-Betainen der Formel (I) als Ausgangssubstanzen benötigten Pyridin-Derivate sind durch die Formel (II) allgemein definiert. In dieser Formel haben X und n die oben angegebenen Bedeutungen.

$R^5$ steht vorzugsweise für Alkyl mit 1 bis 6 Kohlenstoffatomen, insbesondere für Methyl oder Ethyl.

Die Pyridin-Derivate der Formel (II) sind bekannt oder lassen sich nach bekannten Verfahren herstellen (vgl. JP-OS 1987-161 761 und JP-PS 1980-108 818).

Die bei dem obigen Verfahren a) zur Herstellung von Pyridinium-Betainen als Reaktionskomponenten benötigten Verbindungen sind durch die Formel (III) allgemein definiert. In dieser Formel hat R die oben angegebene Bedeutung. Y steht vorzugsweise für Chlor, Brom, Iod, Sulfonyl, Methosulfat oder für den Rest

der Formel $-O^{\oplus}R^6BF_4^{\ominus}$, worin $R^6$ für Methyl oder Ethyl steht.

Die Verbindungen der Formel (III) sind bekannt oder lassen sich nach bekannten Verfahren herstellen.

Als Verdünnngsmittel kommen bei der Durchführung der ersten Stufe des obigen Verfahrens a) alle inerten organischen Solventien in Betracht. Vorzugsweise verwendbar sind aliphatische und aromatische Kohlenwasserstoffe, wie Pentan, Hexan, Benzol, Toluol und Xylol, ferner Ether, wie Diethylether, Dioxan oder Tetrahydrofuran, weiterhin Ester, wie Essigsäureethylester, ferner Nitrile, wie Acetonitril, und außerdem polare organische Lösungsmittel, wie Dimethylformamid oder Dimethylsulfoxid. In manchen Fällen kann auch Wasser, gegebenenfalls im Gemisch mit einem organischen Lösungsmittel als Verdünnungsmittel verwendet werden.

Die Reaktionstemperaturen können bei der Durchführung der ersten Stufe des obigen Verfahrens a) innerhalb eines größeren Bereiches variiert werden. Im allgemeinen arbeitet man bei Temperaturen zwischen 0 °C und 200 °C, vorzugsweise zwischen 20 °C und 150 °C.

Bei der Durchführung der ersten Stufe des obigen Verfahrens a) setzt man auf 1 Mol an Pyridin-Derivat der Formel (II) im allgemeinen 1 bis 10 Mol, vorzugsweise 1 bis 3 Mol an einer Verbindung der Formel (III) ein. Die Aufarbeitung erfolgt nach üblichen Methoden.

Bei der Durchführung der zweiten Stufe des obigen Verfahrens a) nach Variante ($\alpha$) kommen als basische Ionenaustauscher alle üblichen, mit Hydroxylgruppen beladenen Ionenaustauscher in Frage. Vorzugsweise verwendbar sind Ionenaustauscher, die unter der Bezeichnung "Lewatit" im Handel sind.

Als Verdünnungsmittel kommen bei der Durchführung der zweiten Stufe des obigen Verfahrens a) nach Variante ($\alpha$) alle für derartige Zwecke üblichen organischen Solventien sowie Wasser in Betracht. Vorzugsweise verwendbar sind Wasser, Alkohole, wie Methanol oder Ethanol, sowie polare organische Lösungsmittel, wie Dimethylformamid oder Dimethylsulfoxid, ferner auch Wasser im Gemisch mit einem der genannten organischen Solventien.

Bei der Durchführung der zweiten Stufe des obigen Verfahrens a) nach Variante ($\alpha$) geht man im allgemeinen so vor, daß man das Pyridinium-Salz in einer geringen Menge an Solvens gelöst auf den jeweiligen, in einer Säule befindlichen Anionenaustauscher aufträgt und dann eluiert. Aus dem Eluat lassen sich die gewünschten Verbindungen durch Abziehen des Verdünnungsmittels isolieren und gegebenenfalls nach üblichen Methoden reinigen.

Als Verdünnungsmittel kommen bei der Durchführung der zweiten Stufe des obigen Verfahrens a) nach Variante ($\beta$) Wasser sowie alle für derartige Umsetzungen üblichen organischen Solventien in Frage. Vorzugsweise verwendbar sind diejenigen Lösungsmittel, die bereits im Zusammenhang mit der Variante ($\alpha$) als bevorzugt genannt wurden.

Die Temperaturen können bei der Durchführung der zweiten Stufe des obigen Verfahrens a) nach Variante ($\beta$) innerhalb eines größeren Bereiches variiert werden. Im allgemeinen arbeitet man bei Temperaturen zwischen 0 °C und 100 °C, vorzugsweise zwischen 20 °C und 50 °C.

Bei der Durchführung der zweiten Stufe des obigen Verfahrens a) nach Variante ($\beta$) geht man im allgemeinen so vor, daß man auf 1 Mol an Pyridinium-Salz der Formel (IV) im allgemeinen 1 bis 5 Mol, vorzugsweise 1 bis 2 Mol an Silberoxid einsetzt. Die Aufarbeitung erfolgt nach üblichen Methoden.

Die bei dem Verfahren b) zur Herstellung von Pyridinium-Betainen der Formel (I) als Ausgangsstoffe benötigten Pyridincarbonsäuren sind durch die Formel (V) allgemein definiert. In dieser Formel haben X und n die oben angegebenen Bedeutungen.

Die Pyridincarbonsäuren sind bekannt oder lassen sich nach bekannten Methoden herstellen.

Die bei dem Verfahren b) zur Herstellung von Pyridinium-Betainen der Formel (I) als Reaktionskomponenten benötigten Olefine sind durch die Formel (VI) allgemein definiert. In dieser Formel steht $Z^1$ für einen elektronenziehenden Rest der Formel $-COOR^7$, $-CONR^8R^9$, -CN, $-COR^{10}$ oder $-SO_2R^{11}$, worin

| | |
|---|---|
| $R^7$, $R^8$, $R^9$ und $R^{10}$ | unabhängig voneinander vorzugsweise für Wasserstoff, Alkyl mit 1 bis 4 Kohlenstoffatomen, Phenyl, Benzyl oder Cycloalkyl mit 5 oder 6 Kohlenstoffatomen stehen und |
| $R^{11}$ | vorzugsweise für Alkyl mit 1 bis 4 Kohlenstoffatomen, Phenyl, Benzyl oder Cycloalkyl mit 5 oder 6 Kohlenstoffatomen steht. |

Die aktivierten Olefine der Formel (VI) sind bekannt oder lassen sich nach bekannten Methoden herstellen.

Als Verdünnungsmittel kommen bei der Durchführung des Verfahrens b) polare Solventien in Betracht. Vorzugsweise verwendbar sind Wasser, Alkohole, wie Methanol oder Ethanol, oder Carbonsäuren, wie Eisessig. Es ist jedoch auch möglich, ohne Zusatz eines Verdünnungsmittels zu arbeiten.

Die Temperaturen können bei der Durchführung des Verfahrens b) innerhalb eines größeren Bereiches variiert werden. Im allgemeinen arbeitet man bei Temperaturen zwischen 0 °C und 160 °C, vorzugsweise zwischen 20 °C und der Siedetemperatur des jeweiligen Verdünnungsmittels.

Bei der Durchführung des Verfahrens b) geht man so vor, daß man auf 1 Mol an Pyridincarbonsäure der Formel (V) im allgemeinen 1 bis 2 Mol, vorzugsweise 1 Mol an aktiviertem Olefin der Formel (VI) einsetzt. Die Aufarbeitung erfolgt nach üblichen Methoden.

Als Verdünnungsmittel kommen bei der Durchführung des Verfahrens c) alle hochsiedenden inerten Lösungsmittel in Frage. Vorzugsweise arbeitet man jedoch ohne Zusatz eines Verdünnungsmittels.

Die Temperaturen können auch bei der Durchführung des Verfahrens (c) innerhalb eines größeren Bereiches variiert werden. Im allgemeinen arbeitet man bei Temperaturen zwischen 100 °C und 200 °C, vorzugsweise zwischen 140 °C und 180 °C.

Bei der Durchführung des Verfahrens (c) geht man im allgemeinen so vor, daß man das Pyridinium-Derivat der Formel (II) gegebenenfalls in Gegenwart eines Verdünnungsmittels bis zur Beendigung der Umlagerung auf die jeweils gewünschte Temperatur erhitzt. Die Aufarbeitung erfolgt wiederum nach üblichen Methoden.

Die erfindungsgemäß verwendbaren Pyridinium-Betaine weisen eine starke resistenz-induzierende Wirkung in Pflanzen auf. Sie eignen sich daher zur Erzeugung von Resistenz in Pflanzen gegen Befall durch unerwünschte Mikroorganismen.

Unter resistenz-induzierenden Stoffen sind im vorliegenden Zusammenhang solche Substanzen zu verstehen, die einerseits bei direkter Einwirkung auf die unerwünschten Mikroorganismen nur eine geringe Aktivität zeigen, andererseits aber in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäß verwendbaren Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen.

Zu den unerwünschten Mikroorganismen im Pflanzenschutz gehören Pilze aus den Klassen der Plasmodiophoromycetes, Oomycetes, Chytridiomycetes, Zygomycetes, Asomycetes, Basidiomycetes und Deuteromycetes. Beispielhaft, aber nich begrenzend seien einige Erreger von pilzlichen, bakteriellen und virösen Erkrankungen genannt, die unter die oben aufgezählten Oberbegriffe fallen:

Phytophthora-Arten, wie beispielsweise Phytophthora infestans;
Pseudoperonospora-Arten, wie beispielsweise Pseudoperonospora humuli oder Pseudoperonospora cubense;
Plasmonara-Arten, wie beispielsweise Plasmonara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder Pyrenophora graminea (Konidienform; Drechslera sp., Syn. Helminthosporium sp.,);
Cochliobolus-Arten, wie beispielsweise Cochliobolus sativus (Konidienform: Drechslera sp., Syn. Helminthosporium sp);
Uyromyces-Arten, wie beispielsweise Uromyces phaseoli;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum oder Fusarium graminearum;
Botrytis-Arten, wie beispielsweise Botrytis cinerea;
Leotosphaeria-Arten, wie beispielsweise Leptosphaeria nodorum oder Lentosphaeria tritici (Konidienform: Sentoria sp.);
Lentosphaeria-Arten, wie beispielsweise Leptosphaeria maculans (Konidienform: Phoma lingam);
Cercospora-Arten, wie beispielsweise Cercospora canescens oder Cercospora beticola;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zu Induktion von Krankheitsresistenz in Pflanzen notwendigen Konzentrationen erlaubt eine Behandlung von oberirdischen Pflanzenteilen, von Pflanz- und Saatgut und des Bodens.

Dabei lassen sich die erfindungsgemäß verwendbaren Wirkstoffe mit besonders gutem Erfolg einsetzen

- zur Verhinderung des Auftretens von Getreidekrankheiten, wie beispielsweise des Getreidemehltaus (Erysiphe graminis),
  der Streifenkrankheit der Gerste (Pyrenophora graminea),
  der Braunfleckenkrankheit des Getreides (Cochliobolus sativus), der Netzfleckenkrankheit der Gerste (Pyrenophora teres),
  der Septoria-Erkrankung des Getreides (Septoria nodorum, Septoria tritici),
  der Fusarium-Erkrankungen (Fusarium culmorum, Fusarium graminearum),
  der Rostkrankheiten des Getreides (Puccinia recondita, Puccinia graminis, Puccinia glumarum),
- zur Verhinderung des Auftretens von Reiskrankheiten, die beispielsweise hervorgerufen werden durch Pyricularia oryzae oder Pellicularia sasakii,
- zur Verhinderung des Auftretens von Obst- und Gemüsekrankheiten, wie beispielsweise
  den Echten Mehltau an verschiedenen Wirtspflanzen (Erysiphe sp., Sphaerotheca sp., Podosphaera sp.);
  den Falschen Mehltau an verschiedenen Wirtspflanzen (Plasmopara sp., Peronospora sp., Pseudoperonospora sp., Bremia sp.);
  den Schorf an verschiedenen Wirtspflanzen (Venturia sp.);
  den Grauschimmel an verschiedenen Wirtspflanzen (Botrytis cinerea).

Neben der Resistenz-induzierenden Wirkung zeichnen sich die erfindungsgemäß verwendbaren Wirkstoffe insbesondere auch durch eine gute systemische Wirksamkeit aus.

Zu den unerwünschten Mikroorganismen im Pflanzenschutz gehören auch Viren. Als Beispiele genannt seien das Tabakmosaik-Virus, das Apfelmosaik-Virus und das Gurkenmosaik-Virus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feistverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigteen Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Tragerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Bakteriziden, Akariziden, Nematiziden, Herbiziden, Schutzstoffen gegen Vogelfraß, Wuchsstoffen, Pflanzennährstoffen und Bodenstrukturverbesserungsmitteln.

Als Beispiele für Fungizide seien in diesem Zusammenhang genannt: Imazalil, Benomyl, Carbendazim, Thiophanatmethyl, Captafol, Captan, Schwefel, Triforin, Dodemorph, Tridemorph, Pyrazophos, Furalaxyl, Ethirimol, Dimethirimol, Bupirimat, Chlorothalonil, Vinclozolin, Procymidon, Iprodion, Metalaxyl, Carboxin, Oxycarboxin, Fenarimol, Nuarimol, Fenfuram, Methfuroxan, Nitrotal-isopropyl, Triadimefon, Thiabendazol, Etridiazol, Triadimenol, Bitertanol, Propiconazol, Anilazin, Tebuconazol, Biloxazol, Dithianon, Binapacryl, Quinomethionat, Guazatin, Dodine, Fentin-acetat, Fentinhydroxid, Dinocap, Folpet, Dichlofluanid, Ditalimphos, Kitazin, Cycloheximid, Dichlobutrazol, Fenapanil, Ofurace, Etaconazol und Fenpropemorph.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Anwendung als Resistenzinduktoren können die Wirkstoffkonzentrationen für die Behandlung von Pflanzenteilen in den Anwendungsformen in einem größeren Bereich variiert werden. Sie liegen im allgemeinen zwischen 1 und 0,0001 Gewichtsprozent, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 1,0 Gewichtsprozent, vorzugsweise 0,0001 bis 0,02 Gewichtsprozent, am Wirkort erforderlich.

Die Herstellung und die Verwendung der erfindungsgemäßen Stoffe wird durch die folgenden Beispiele veranschaulicht.

Herstellungsbeispiele

Bespiel 1

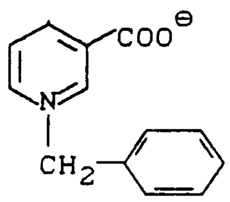

(I-1)

Zu einer Lösung von 22,7 g (0,165 Mol) Nicotinsäuremethylester in 250 ml Toluol werden 30,9 g (0,182 Mol) Benzylbromid gegeben. Das Reaktionsgemisch wird 16 Stunden unter Rückfluß erhitzt und dann abgekühlt. Der dabei anfallende Teststoff wird abfiltriert und getrocknet. Man erhält auf diese Weise 24,1 g (48% der Theorie) an 1-Benzyl-3-carbomethoxy-pyridinium-bromid in Form einer Festsubstanz.

NMR-Spektrum; 400 MHz
($DMSO-d_6$/TMS)

| | |
|---|---|
| δ 4,0 ppm | $COOC\underline{H_3}$ |
| δ 6,1 ppm | $N-\underline{CH_2}-$ 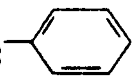 |
| δ 8,4-8,5 ppm | 1H-Pyridin |

Eine Lösung von 10 g (32,6 mMol) 1-Benzyl-3-carbomethoxy-pyridinium-bromidin 100 ml Wasser wird auf eine mit einem Ionenaustauscher der Bezeichnung "Lewatit MP 500" gefüllte Chromatographie-Säule (Länge: 20 cm; Durchmesser: 3 cm) gegeben. Man eluiert mit 250 ml Wasser. Nach dem Einengen des aufgefangenen Eluates unter vermindertem Druck verbleibt ein hellgelber Kristallbrei, der mit 20 ml Ethanol versetzt wird. Der Feststoff wird abfiltriert, und das Filtrat wird mit 20 ml Diethylether versetzt. Das sich dabei abscheidende Produkt wird abfiltriert und mit dem zuvor abfiltrierten Feststoff vereinigt. Man erhält auf diese Weise 5,0 g (72% der Theorie) an 1-Benzyl-3-carboxy-pyridiniumbetain in Form eines farblosen Feststoffes vom Schmelzpunkt 206 °C.

Nach der im Beispiel 1 angegebenen Methode werden auch die in der folgenden Tabelle 4 aufgeführten Pyridinium-Betaine der Formel (I) hergestellt.

Tabelle 4

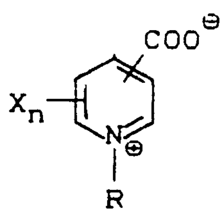

(I)

| Beispiel Nr. | Verbindung Nr. | R | $X_n$ | Position der $COO^{\ominus}$ Gruppe | Physikalische Konstante |
|---|---|---|---|---|---|
| 2 | I-2 | $-C_2H_5$ | - | 3 | Fp=150° C |
| 3 | I-3 | $-CH_3$ | - | 3 | Fp=220° C |
| 4 | I-4 | $-CH_3$ | - | 4 | NMR δ=4,45 ppm |
| 5 | I-5 | $-C_2H_5$ | - | 4 | NMR δ=4,7-4,8 ppm |
| 6 | I-6 | $-CH_2-$ 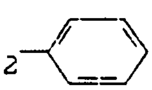 | - | 4 | NMR δ=5,8 ppm |
| 7 | I-7 | $-CH_3$ | - | 2 | Fp=170-173° C |
| 8 | I-8 | $-C_2H_5$ | - | 2 | Fp=121-124° C |

Beispiel 9

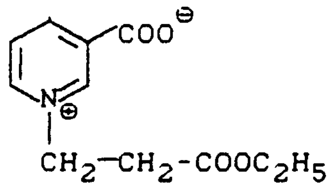

(I-9)

Ein Gemisch aus 6,2 g (0,05 Mol) Nicotinsäure und 10 g (0,1 Mol) Acrylsäureethylester in 100 ml Eisessig wird 20 Stunden unter Rückfluß erhitzt. Danach wird das Verdünnungsmittel abdestilliert. Der verbleibende Rückstand wird zur Abtrennung von noch enthaltener Essigsäure und Nicotinsäure in 60 ml Wasser gelöst. Die Lösung wird auf eine Säule, die mit 150 ml des schwach basischen Ionenaustauschers Lewatit MP62 gefüllt ist, aufgetragen und mit 500 ml Wasser eluiert. Das Eluat wird gefriergetrocknet und aus einem Gemisch von Ethanol:Essigester = 1:1 umkristallisiert. Man erhält auf diese Weise 5,95 g (53 % der Theorie) an 3-Carboxy-1-[2-(ethoxycarbonyl)-ethyl]-pyridiniumbetain in Form eines farblosen Feststoffes vom Schmelzpunkt 108 - 110 °C.

Nach der im Beispiel 9 angegebenen Methode werden auch die in den folgenden Beispielen aufgeführten Pyridinium-Betaine hergestellt.

Beispiel 10

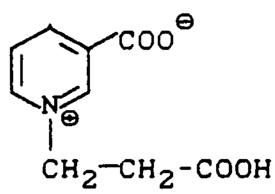

(I-10)

Fp = 174-176 °C

Beispiel 11

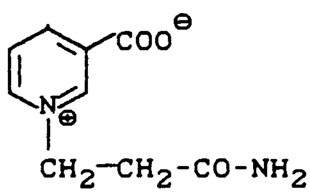

(I-11)

Fp = 222-223 °C

In den folgenden Verwendungsbeispielen wird die Verbindung der nachstehend angegebenen Formel als Vergleichssubstanz eingesetzt:

(A) =

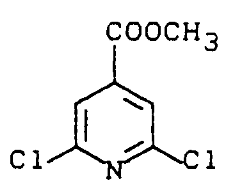

(Bekannt aus EP-OS 0 268 775)

Beispiel A

Plasmopara-Test an Blattstücken von Reben / protektiv (Resistenzinduktion)

| | |
|---|---|
| Lösungsmittel: | 4,7 Gewichtsteile Aceton |
| Emulgator: | 0,3 Gewichtsteile Alkyl-arylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung der Resistenz-induzierenden Wirksamkeit bespritzt man isolierte Blätter/Blattstücke mit der Wirkstoffzubereitung bis zur Tropfnässe. Nach einer Vorbehandlungsdauer von 3 Tagen werden die Pflanzen mit einer wäßrigen Sporensuspension von Plasmopara viticola inokuliert. Isolierte Blätter bzw. Blattstücke werden in geeigneten, belichteten Kammern bei 20 bis 22 °C und hoher Luftfeuchte (95-100%) für 6 Tage inkubiert. Anschließend werden die Pflanzen für 1 Tag bei 100% relativer Luftfeuchte aufgestellt.

Die Auswertung erfolgt 7 Tage nach der Inokulation.

Wirkstoffe, Wirkstoffkonzentrationen und Versuchsergebnisse gehen aus der folgenden Tabelle hervor.

Tabelle A

Plasmopara-Test an Blattstücken von Reben/protektiv (Resistenzinduktion)

| Wirkstoff | Wirkungsgrad in % der unbehandelten Kontrolle bei einer Wirkstoffkonzentration von 100 ppm |
|---|---|
| - (Kontrolle) | = 0 |
| Bekannt: | |
| 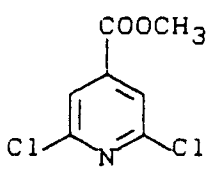 (A) | 40 |
| Erfindungsgemäß: | |
| 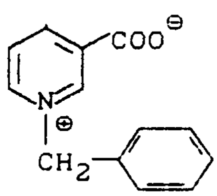 (I-1) | 75 |
| 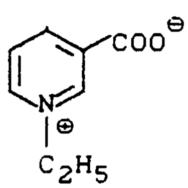 (I-2) | 85 |

Tabelle A (Fortsetzung)

Plasmopara-Test an Blattstücken von Reben/protektiv (Resistenzinduktion)

| Wirkstoff | Wirkungsgrad in % der unbehandelten Kontrolle bei einer Wirkstoffkonzentration von 100 ppm |
|---|---|
| 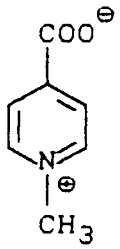 (I-3) | 75 |

Beispiel B

Botrytis-Test an Blattstücken von Reben/protektiv (Resistenzinduktion)

| | |
|---|---|
| Lösungsmittel: | 4,7 Gewichtsteile Aceton |
| Emulgator: | 0,3 Gewichtsteile Alkyl-arylpolyglykolether |

Zur Herstellung einer zweckmäßigen Wirkstoffzubereitung vermischt man 1 Gewichtstell Wirkstoff mit den angegebenen Mengen Lösungsmittel und Emulgator und verdünnt das Konzentrat mit Wasser auf die gewünschte Konzentration.

Zur Prüfung der Resistenz-induzierenden Wirksamkeit bespritzt man isolierte Blätter/Blattstücke mit der Wirkstoffzubereitung bis zur Tropfnässe. Nach einer Vorbehandlungsdauer von 3 Tagen werden die Pflanzen mit einer wäßrigen Sporensuspension von Botrytis cinerea inokuliert. Die isolierten Pflanzenteile (Blätter bzw. Blattstücke) werden in geeigneten, belichteten Kammern bei 20 bis 22°C und hoher Luftfeuchte (95-100%) inkubiert.

Am 3. Tag nach der Inokulation wird die Größe der Befallsflecken auf den Blättern ausgewertet und der Wirkungsgrad bestimmt.

Wirkstoffe, Wirkstoffkonzentrationen und Versuchsergebnisse gehen aus der folgenden Tabelle hervor.

Tabelle B

Botrytis-Test an Blattstücken von Reben/protektiv (Resistenzinduktion)

| Wirkstoff | Wirkungsgrad in % der unbehandelten Kontrolle bei einer Wirkstoffkonzentration von 100 ppm |
|---|---|
| - (Kontrolle) | = 0 |
| Bekannt: | |
| 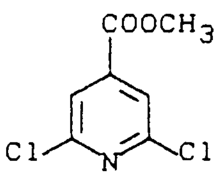 (A) | 26 |
| Erfindungsgemäß: | |
| 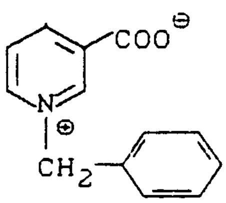 (I-1) | 43 |
| 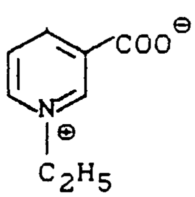 (I-2) | 49 |

Tabelle B (Fortsetzung)

Botrytis-Test an Blattstücken von Reben/protektiv (Resistenzinduktion)

| Wirkstoff | Wirkungsgrad in % der unbehandelten Kontrolle bei einer Wirkstoffkonzentration von 100 ppm |
|---|---|
| 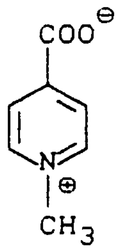 (I-3) | 89 |

Beispiel C

Phytophthora-Test an Blattstücken von Tomaten/protektiv (Resistenzinduktion)

| | |
|---|---|
| Lösungsmittel: | 4,7 Gewichtsteile Aceton |
| Emulgator: | 0,3 Gewichtsteile Alkyl-arylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung der Resistenz-induzierenden Wirksamkeit bespritzt man isolierte Blätter/Blattstücke mit der Wirkstoffzubereitung bis zur Tropfnasse. Nach einer Vorbehandlungsdauer von 3 Tagen werden die Pflanzen mit einer wäßrigen Sporensuspension von Phytophthora infestans inokuliert. Die isolierten Pflanzenteile (Blätter bzw. Blattstücke) werden in geeigneten, belichteten Kammern bei 20 bis 22 °C und hoher Luftfeuchte (95-100%) inkubiert.

Am 3. Tag nach der Inokulation wird die Größe der Befallsflecken auf den Blättern ausgewertet und der Wirkungsgrad bestimmt.

Wirkstoffe, Wirkstoffkonzentrationen und Versuchsergebnisse gehen aus der folgenden Tabelle hervor.

Tabelle C

Phytophthora-Test an Blattstücken von Tomaten/protektiv (Resistenzinduktion)

| Wirkstoff | Wirkungsgrad in % der unbehandelten Kontrolle bei einer Wirkstoffkonzentration von 100 ppm |
|---|---|
| - (Kontrolle) | = 0 |
| **Bekannt:** | |
| 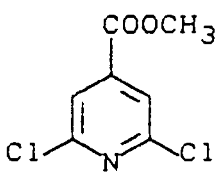 (A) | 47 |
| **Erfindungsgemäß:** | |
| 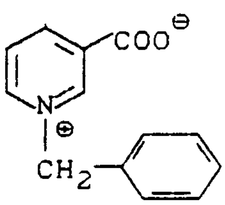 (I-1) | 100 |
| 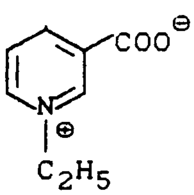 (I-2) | 100 |

Tabelle C (Fortsetzung)

Phytophthora-Test an Blattstücken von Tomaten/protektiv (Resistenzinduktion)

| Wirkstoff | Wirkungsgrad in % der unbehandelten Kontrolle bei einer Wirkstoffkonzentration von 100 ppm |
|---|---|
| 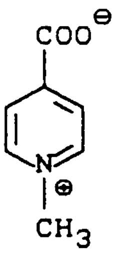 (I-3) | 100 |

## Patentansprüche

1. Verwendung von Pyridinium-Betainen der Formel

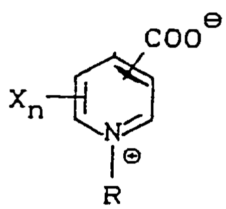

(I)

in welcher

R für Alkyl mit 1 bis 8 Kohlen stoffatomen steht, wobei die Alkylreste einfach bis dreifach, gleichartig oder verschieden substituiert sein können durch Halogen, Hydroxy, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Cyano, Trimethylsilyl, gegebenenfalls durch Halogen und/oder Alkyl mit 1 bis 4 Kohlenstoffatomen substituiertes Cycloalkyl mit 3 bis 6 Kohlenstoffatomen, gegebenenfalls durch Halogen und/oder Alkyl mit 1 bis 4 Kohlenstoffatomen substituiertes Cycloalkenyl mit 5 oder 6 Kohlenstoffatomen, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylthio,

sowie die Reste der Formeln

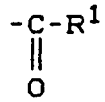 , 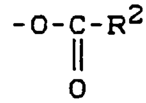, 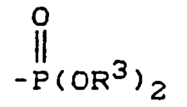 und

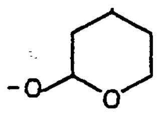 ,

worin

$R^1$ für Alkyl mit 1 bis 6 Kohlenstoffatomen, gegebenenfalls durch Halogen, Alkyl mit 1 bis 4 Kohlenstoffatomen und/oder Alkoxy mit 1 bis 4 Kohlenstoffatomen substituiertes Phenyl, Hydroxy, Alkoxy mit 1 bis 14 Kohlenstoffatomen, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Benzyloxy, Amino, Alkylamino mit 1 bis 4 Kohlenstoffatomen, Dialkylamino mit 1 bis 4 Kohlenstoffatomen in jeder Alkylgruppe, gegebenenfalls durch Halogen und/oder Alkyl mit 1 bis 4 Kohlenstoffatomen substituiertes Phenyl oder den Rest der Formel $-NH-CH_2-CO-R^4$ steht, worin

$R^4$ für Hydroxy, Alkoxy mit 1 bis 4 Kohlenstoffatomen, Alkylamino mit 1 bis 4 Kohlenstoffatomen oder für gegebenenfalls durch Halogen und/oder Alkyl mit 1 bis 4 Kohlenstoffatomen substituiertes Phenylamino steht,

$R^2$ für Alkyl mit 1 bis 20 Kohlenstoffatomen, Alkylamino mit 1 bis 4 Kohlenstoffatomen, Dialkylamino mit 1 bis 4 Kohlenstoffatomen in jeder Alkylgruppe oder für gegebenenfalls durch Halogen und/oder Alkyl mit 1 bis 4 Kohlenstoffatomen substituiertes Phenylamino steht und

$R^3$ für Alkyl mit 1 bis 4 Kohlenstoffatomen steht,

oder

R für Alkenyl mit 3 bis 8 Kohlenstoffatomen steht, wobei jeder der Alkenylreste einfach bis dreifach, gleichartig oder verschieden substituiert sein kann durch Halogen, Alkoxy mit 1 bis 4 Kohlenstoffatomen und/oder Alkylthio mit 1 bis 4 Kohlenstoffatomen, oder

R für Alkinyl mit 3 bis 8 Kohlenstoffatomen steht, wobei jeder der Alkinylreste einfach bis dreifach, gleichartig oder verschieden substituiert sein kann durch Halogen, Alkoxy mit 1 bis 4 Kohlenstoffatomen und/oder Alkylthio mit 1 bis 4 Kohlenstoffatomen, oder

R für Aralkyl mit 6 bis 10 Kohlenstoffatomen im Arylteil und 1 bis 4 Kohlenstoffatomen im Alkylteil steht, wobei jeder dieser Reste im Arylteil einfach bis dreifach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R für Aralkenyl mit 6 bis 10 Kohlenstoffatomen im Arylteil und 2 bis 4 Kohlenstoffatomen im Alkenylteil steht, wobei jeder dieser Reste im Arylteil einfach bis dreifach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R für Aralkinyl mit 6 bis 10 Kohlenstoffatomen im Arylteil und 2 bis 4 Kohlenstoffatomen im Alkinylteil steht, wobei jeder dieser Reste im Arylteil einfach bis dreifach, gleichartig oder verschieden substituiert sein kann durch Halogen, Alkyl mit 1 bis 4 Kohlenstoffatomen,

Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro, Cyano, Phenyl, Phenoxy, Phenylthio, Alkoxycarbonyl mit 1 bis 4 Kohlenstoffatomen im Alkoxyteil und/oder Alkylcarbonyl mit 1 bis 4 Kohlenstoffatomen im Alkylteil, oder

R steht vorzugsweise für gegebenenfalls einfach bis dreifach, gleichartig oder verschieden durch Alkyl mit 1 bis 4 Kohlenstoffatomen substituiertes Cycloalkyl mit 3 bis 8 Kohlenstoffatomen steht, oder

R für gegebenenfalls einfach bis dreifach, gleichartig oder verschieden durch Alkyl mit 1 bis 4 Kohlenstoffatomen substituiertes Cycloalkenyl mit 5 bis 8 Kohlenstoffatomen steht, oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Nitro und/oder Cyano substituiertes Heterocyclylalkyl mit 1 bis 4 Kohlenstoffatomen im Alkylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,
oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Nitro und/oder Cyano substituiertes Heterocyclylalkenyl mit 3 bis 6 Kohlenstoffatomen im Alkenylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,
oder

R für gegebenenfalls einfach bis dreifach, gleichartig oder verschieden durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen, Nitro und/oder Cyano substituiertes Heterocyclylalkinyl mit 3 bis 6 Kohlenstoffatomen im Alkinylteil und 5 oder 6 Ringgliedern im Heterocyclus steht, wobei der Heterocyclus gesättigt oder ungesättigt sein kann und 1 bis 3 Heteroatome, wie Sauerstoff, Schwefel und/oder Stickstoff, sowie 1 oder 2 Carbonylgruppen enthalten kann und auch einen anellierten Benzolrest enthalten kann,

X für Fluor, Chlor, Brom, Jod, Alkyl mit 1 bis 8 Kohlenstoffatomen, Halogenalkyl mit 1 bis 4 Kohlenstoffatomen und 1 bis 5 gleichen oder verschiedenen Halogenatomen, Alkoxy mit 1 bis 8 Kohlenstoffatomen, Halogenalkoxy mit 1 bis 4 Kohlenstoffatomen und 1 bis 5 gleichen oder verschiedenen Halogenatomen, Hydroxy, Mercapto, Alkylthio mit 1 bis 8 Kohlenstoffatomen, Halogenalkylthio mit 1 bis 4 Kohlenstoffatomen und 1 bis 5 gleichen oder verschiedenen Halogenatomen, Amino, Alkylamino mit 1 bis 6 Kohlenstoffatomen, Dialkylamino mit 1 bis 6 Kohlenstoffatomen in jeder Alkylgruppe, Phenylamino, Benzylamino oder gegebenenfalls durch Halogen und/oder Alkyl mit 1 bis 4 Kohlenstoffatomen substituiertes Phenyl steht,
und

n für 0, 1, 2, 3 oder 4 steht,

wobei die durch X bezeichneten Substituenten gleichartig oder verschieden sein können, wenn n für 2, 3 oder 4 steht,
zur Erzeugung von Resistenz in Pflanzen gegen Befall durch unerwünschte Mikroorganismen.

## Claims

1. Use of pyridinium betaines of the formula

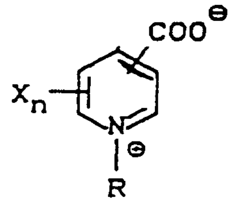

(I)

in which

R represents alkyl having 1 to 8 carbon atoms, it being possible for the alkyl radicals to be monosubstituted to trisubstituted by identical or different substituents from the series consisting of halogen, hydroxyl, alkoxy having 1 to 4 carbon atoms, alkylthio having 1 to 4 carbon atoms, alkylsulphinyl having 1 to 4 carbon atoms, alkylsulphonyl having 1 to 4 carbon atoms, cyano, trimethylsilyl, cycloalkyl having 3 to 6 carbon atoms which is optionally substituted by halogen and/or alkyl having 1 to 4 carbon atoms, cycloalkenyl having 5 or 6 carbon atoms which is optionally substituted by halogen and/or alkyl having 1 to 4 carbon atoms, phenoxy which is optionally substituted by halogen and/or alkyl having 1 to 4 carbon atoms, phenylthio which is optionally substituted by halogen and/or alkyl having 1 to 4 carbon atoms, and the radicals of the formulae

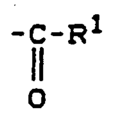 , 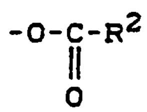 , 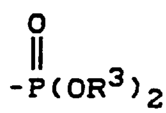 and

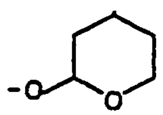 ,

in which

$R^1$ represents alkyl having 1 to 6 carbon atoms, phenyl which is optionally substituted by halogen, alkyl having 1 to 4 carbon atoms and/or alkoxy having 1 to 4 carbon atoms, or hydroxyl, alkoxy having 1 to 14 carbon atoms, phenoxy which is optionally substituted by halogen and/or alkyl having 1 to 4 carbon atoms, benzyloxy which is optionally substituted by halogen and/or alkyl having 1 to 4 carbon atoms, or amino, alkylamino having 1 to 4 carbon atoms, dialkylamino having 1 to 4 carbon atoms in each alkyl group, phenyl which is optionally substituted by halogen and/or alkyl having 1 to 4 carbon atoms, or the radical of the formula $-NH-CH_2-CO-R^4$, in which

$R^4$ represents hydroxyl, alkoxy having 1 to 4 carbon atoms, alkylamino having 1 to 4 carbon atoms, or phenylamino which is optionally substituted by halogen and/or alkyl having 1 to 4 carbon atoms,

$R^2$ represents alkyl having 1 to 20 carbon atoms, alkylamino having 1 to 4 carbon atoms, dialkylamino having 1 to 4 carbon atoms in each alkyl group, or phenylamino which is optionally substituted by halogen and/or alkyl having 1 to 4 carbon atoms, and

$R^3$ represents alkyl having 1 to 4 carbon atoms,

or

R represents alkenyl having 3 to 8 carbon atoms, it being possible for each of the alkenyl radicals to be monosubstituted to trisubstituted by identical or different substituents from the series consisting of halogen, alkoxy having 1 to 4 carbon atoms and/or alkylthio having 1 to 4 carbon atoms, or

R represents alkinyl having 3 to 8 carbon atoms, it being possible for each of the alkinyl radicals to be monosubstituted to trisubstituted by identical or different substituents from the series consisting of halogen, alkoxy having 1 to 4 carbon atoms and/or alkylthio having 1 to 4 carbon atoms, or

R represents aralkyl having 6 to 10 carbon atoms in the aryl moiety and 1 to 4 carbon atoms in the alkyl moiety, it being possible for each of these radicals to be monosubstituted to trisubstituted in the aryl moiety by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, alkylthio having 1 to 4 carbon atoms, halogenoalkylthio having 1 to 4 carbon atoms and 1 to 5 identical or different halogen atoms, nitro, cyano, phenyl, phenoxy, phenylthio, alkoxycarbonyl having 1 to 4 carbon atoms in the alkoxy moiety and/or alkylcarbonyl having 1 to 4 carbon atoms in the alkyl moiety, or

R represents aralkenyl having 6 to 10 carbon atoms in the aryl moiety and 2 to 4 carbon atoms in the alkenyl moiety, it being possible for each of these radicals to be monosubstituted to trisubstituted in the aryl moiety by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, alkylthio having 1 to 4 carbon atoms, halogenoalkylthio having 1 to 4 carbon atoms and 1 to 5 identical or different halogen atoms, nitro, cyano, phenyl, phenoxy, phenylthio, alkoxycarbonyl having 1 to 4 carbon atoms in the alkoxy moiety and/or alkylcarbonyl having 1 to 4 carbon atoms in the alkyl moiety, or

R represents aralkinyl having 6 to 10 carbon atoms in the aryl moiety and 2 to 4 carbon atoms in the alkinyl moiety, it being possible for each of these radicals to be monosubstituted to trisubstituted in the aryl moiety by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, alkylthio having 1 to 4 carbon atoms, halogenoalkylthio having 1 to 4 carbon atoms and 1 to 5 identical or different halogen atoms, nitro, cyano, phenyl, phenoxy, phenylthio, alkoxycarbonyl having 1 to 4 carbon atoms in the alkoxy moiety and/or alkylcarbonyl having 1 to 4 carbon-atoms in the alkyl moiety, or

R preferably represents cycloalkyl having 3 to 8 carbon atoms which is optionally monosubstituted to trisubstituted by identical or different substituents from the series consisting of alkyl having 1 to 4 carbon atoms, or

R represents cycloalkenyl having 5 to 8 carbon atoms which is optionally monosubstituted to trisubstituted by identical or different substituents from the series consisting of alkyl having 1 to 4 carbon atoms, or

R represents heterocyclylalkyl which has 1 to 4 carbon atoms in the alkyl moiety and 5 or 6 ring members in the heterocycle and which is optionally monosubstituted to trisubstituted by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, nitro and/or cyano, it being possible for the heterocycle to be saturated or unsaturated and to contain 1 to 3 hetero atoms, such as oxygen, sulphur and/or nitrogen, and 1 or 2 carbonyl groups and also a fused benzene radical,

or

R represents heterocyclylalkenyl which has 3 to 6 carbon atoms in the alkenyl moiety and 5 or 6 ring members in the heterocycle and which is optionally monosubstituted to trisubstituted by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, nitro and/or cyano, it being possible for the heterocycle to be saturated or unsaturated and to contain 1 to 3 hetero atoms, such as oxygen, sulphur and/or nitrogen, and 1 or 2 carbonyl groups and also a fused benzene

radical,

or

R represents heterocyclylalkinyl which has 3 to 6 carbon atoms in the alkinyl moiety and 5 or 6 ring members in the heterocycle and which is optionally monosubstituted to trisubstituted by identical or different substituents from the series consisting of halogen, alkyl having 1 to 4 carbon atoms, halogenoalkyl having 1 to 4 carbon atoms and 1 to 5 identical or different halogen atoms, alkoxy having 1 to 4 carbon atoms, halogenoalkoxy having 1 to 4 carbon atoms and 1 to 5 identical or different halogen atoms, nitro and/or cyano, it being possible for the heterocycle to be saturated or unsaturated and to contain 1 to 3 hetero atoms, such as oxygen, sulphur and/or nitrogen, and 1 or 2 carbonyl groups and also a fused benzene radical,

X represents fluorine, chlorine, bromine, iodine, alkyl having 1 to 8 carbon atoms, halogenoalkyl having 1 to 4 carbon atoms and 1 to 5 identical or different halogen atoms, alkoxy having 1 to 8 carbon atoms, halogenoalkoxy having 1 to 4 carbon atoms and 1 to 5 identical or different halogen atoms, hydroxyl, mercapto, alkylthio having 1 to 8 carbon atoms, halogenoalkylthio having 1 to 4 carbon atoms and 1 to 5 identical or different halogen atoms, amino, alkylamino having 1 to 6 carbon atoms, dialkylamino having 1 to 6 carbon atoms in each alkyl group, phenylamino, benzylamino or phenyl which is optionally substituted by halogen and/or alkyl having 1 to 4 carbon atoms,

and

n represents 0, 1, 2, 3 or 4,

it being possible for the substituents designated by X to be identical or different if n represents 2, 3 or 4,

for generating resistance in plants to infestation by undesired microorganisms.

**Revendications**

**1.** Utilisation de pyridinium-bétaïnes de formule

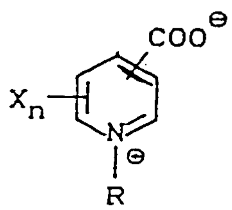

(I)

dans laquelle

R est un reste alkyle ayant 1 à 8 atomes de carbone, les restes alkyle pouvant porter un à trois substituants, identiques ou différents, halogéno, hydroxy, alkoxy ayant 1 à 4 atomes de carbone, alkylthio ayant 1 à 4 atomes de carbone, alkylsulfinyle ayant 1 à 4 atomes de carbone, alkylsulfonyle ayant 1 à 4 atomes de carbone, cyano, triméthylsilyle, cycloalkyle de 3 à 6 atomes de carbone éventuellement substitué par un radical halogéno et/ou alkyle ayant 1 à 4 atomes de carbone, cycloalcényle de 5 ou 6 atomes de carbone substitué le cas échéant par un radical halogéno et/ou alkyle ayant 1 à 4 atomes de carbone, phénoxy substitué le cas échéant par un radical halogéno et/ou alkyle ayant 1 à 4 atomes de carbone, phénylthio substitué le cas échéant par un radical halogéno et/ou alkyle ayant 1 à 4 atomes de carbone, ainsi que les restes de formules

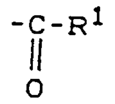 , 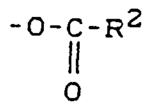, 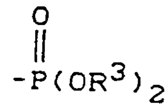 et

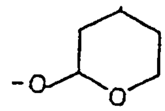

dans lesquelles

R[1] est un groupe alkyle ayant 1 à 6 atomes de carbone, un groupe phényle substitué le cas échéant par un radical halogéno, alkyle ayant 1 à 4 atomes de carbone et/ou alkoxy ayant 1 à 4 atomes de carbone, un groupe hydroxy, alkoxy ayant 1 à 14 atomes de carbone, phénoxy éventuellement substitué par un radical halogéno et/ou alkyle ayant 1 à 4 atomes de carbone, benzyloxy éventuellement substitué par un radical halogéno et/ou alkyle ayant 1 à 4 atomes de carbone, amino, alkylamino ayant 1 à 4 atomes de carbone, dialkylamino ayant 1 à 4 atomes de carbone par groupe alkyle, phényle substitué le cas échéant par un radical halogéno et/ou alkyle ayant 1 à 4 atomes de carbone, ou le reste de formule $-NH-CH_2-CO-R^4$, dans laquelle

$R^4$ est un groupe hydroxy, alkoxy ayant 1 à 4 atomes de carbone, alkylamino ayant 1 à 4 atomes de carbone ou phénylamino éventuellement substitué par un halogène et/ou par un radical alkyle ayant 1 à 4 atomes de carbone,

$R^2$ est un groupe alkyle ayant 1 à 20 atomes de carbone, alkylamino ayant 1 à 4 atomes de carbone, dialkylamino ayant 1 à 4 atomes de carbone dans chaque groupe alkyle ou phénylamino éventuellement substitué par un halogène et/ou un radical alkyle ayant 1 à 4 atomes de carbone et

$R^3$ est un groupe alkyle ayant 1 à 4 atomes de carbone,

ou bien

R est un reste alcényle ayant 3 à 8 atomes de carbone, chacun des restes alcényle pouvant être substitué une à trois fois identiques ou différentes par un halogène, un radical alkoxy ayant 1 à 4 atomes de carbone et/ou un radical alkylthio ayant 1 à 4 atomes de carbone, ou bien

R est un reste alcynyle de 3 à 8 atomes de carbone, chacun des restes alcynyle pouvant être substitué une à trois fois identiques ou différentes par un halogène, un radical alkoxy ayant 1 à 4 atomes de carbone et/ou un radical alkylthio ayant 1 à 4 atomes de carbone, ou bien

R est un reste aralkyle de 6 à 10 atomes de carbone dans la partie aryle et 1 à 4 atomes de carbone dans la partie alkyle, chacun de ces restes pouvant être substitué dans la partie aryle une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone et 1 à 5 atomes d'halogènes identiques ou différents, alkylthio ayant 1 à 4 atomes de carbone, halogénalkylthio ayant 1 à 4 atomes de carbone et 1 à 5 atomes d'halogènes identiques ou différents, nitro, cyano, phényle, phénoxy, phénylthio, alkoxycarbonyle ayant 1 à 4 atomes de carbone dans la partie alkoxy et/ou alkylcarbonyle ayant 1 à 4 atomes de carbone dans la partie alkyle, ou bien

R représente un reste aralcényle ayant 6 à 10 atomes de carbone dans la partie aryle et 2 à 4 atomes de carbone dans la partie alcényle, chacun de ces restes pouvant être substitué dans la partie aryle une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone et 1 à 5 atomes d'halogènes identiques ou différents, alkylthio ayant 1 à 4 atomes de carbone, halogénalkylthio ayant 1 à 4 atomes de carbone et 1 à 5 atomes d'halogènes identiques ou différents, nitro, cyano, phényle, phénoxy, phénylthio, alkoxycarbonyle ayant 1 à 4 atomes de carbone dans la partie alkoxy et/ou alkylcarbonyle ayant 1 à 4 atomes de carbone dans la partie alkyle, ou bien

R est un reste aralcynyle ayant 6 à 10 atomes de carbone dans la partie aryle et 2 à 4 atomes de carbone dans la partie alcynyle, chacun de ces restes pouvant être substitué dans la partie aryle une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone et 1 à 5 atomes d'halogènes identiques ou différents, alkylthio ayant 1 à 4 atomes de carbone, halogénalkylthio ayant 1 à 4 atomes de carbone et 1 à 5 atomes d'halogènes identiques ou différents, nitro, cyano, phényle, phénoxy, phénylthio, alkoxycarbonyle ayant 1 à 4 atomes de carbone dans la partie alkoxy, et/ou alkylcarbonyle ayant 1 à 4 atomes de carbone dans la partie alkyle, ou bien

R représente de préférence un reste cycloalkyle de 3 à 8 atomes de carbone substitué le cas échéant une à trois fois identiques ou différentes par un radical alkyle ayant 1 à 4 atomes de carbone, ou bien

R représente un reste cycloalcényle de 5 à 8 atomes de carbone substitué le cas échéant une à trois fois identiques ou différentes par un radical alkyle ayant 1 à 4 atomes de carbone, ou bien

R représente un reste hétérocyclylalkyle ayant 1 à 4 atomes de carbone dans la partie alkyle et 5 ou 6 chaînons dans l'hétérocycle, substitué le cas échéant une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone et 1 à 5 atomes d'halogènes identiques ou différents, nitro et/ou cyano, l'hétérocycle pouvant être saturé ou non saturé et pouvant contenir 1 à 3 hétéroatomes tels qu'oxygène, soufre et/ou azote ainsi qu'un ou deux groupes carbonyle, et pouvant aussi contenir un reste benzénique condensé,
ou bien

R représente un reste hétérocyclylalcényle de 3 à 6 atomes de carbone dans la partie alcényle et 5 ou 6 chaînons dans l'hétérocycle, substitué le cas échéant une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone et 1 à 5 atomes d'halogènes identiques ou différents, nitro et/ou cyano, l'hétérocycle pouvant être saturé ou non saturé et pouvant contenir 1 à 3 hétéroatomes tels qu'oxygène, soufre et/ou azote ainsi qu'un ou deux groupes carbonyle et pouvant aussi contenir un reste benzénique condensé,
ou

R est un groupe hétérocyclylalcynyle de 3 à 6 atomes de carbone dans la partie alcynyle et 5 ou 6 chaînons dans l'hétérocycle, substitué le cas échéant une à trois fois identiques ou différentes par un halogène, un radical alkyle ayant 1 à 4 atomes de carbone, halogénalkyle ayant 1 à 4 atomes de carbone et 1 à 5 atomes d'halogènes identiques ou différents, alkoxy ayant 1 à 4 atomes de carbone, halogénalkoxy ayant 1 à 4 atomes de carbone, nitro et/ou cyano, l'hétérocycle pouvant être saturé ou non saturé et pouvant contenir 1 à 3 hétéroatomes tels qu'oxygène, soufre et/ou azote ainsi que 1 ou 2 groupes carbonyle et pouvant aussi contenir un reste benzénique condensé,

X représente du fluor, du chlore, du brome, de l'iode, un reste alkyle ayant 1 à 8 atomes de carbone, halogénalkyle ayant 1 à 4 atomes de carbone et 1 à 5 atomes d'halogènes identiques ou différents, alkoxy ayant 1 à 8 atomes de carbone, halogénalkoxy ayant 1 à 4 atomes de carbone et 1 à 5 atomes d'halogènes identiques ou différents, hydroxy, mercapto, alkylthio ayant 1 à 8 atomes de carbone, halogénalkylthio ayant 1 à 4 atomes de carbone et 1 à 5 atomes d'halogènes identiques ou différents, amino, alkylamino ayant 1 à 6 atomes de carbone, dialkylamino ayant 1 à 6 atomes de carbone dans chaque groupe alkyle, phénylamino, benzylamino ou phényle substitué le cas échéant par un halogène et/ou un radical alkyle ayant 1 à 4 atomes de carbone,
et

n a la valeur 0, 1, 2, 3 ou 4,

les substituants représentés par X pouvant être identiques ou différents, lorsque n a la valeur 2, 3 ou 4, pour rendre des plantes aptes à résister à l'attaque par des micro-organismes indésirables.